# EUROPEAN PATENT APPLICATION

(11) **EP 1 336 618 A1**
(43) Date of publication of application: **20.08.2003**
(21) Application number: 02003482.3
(22) Date of filing: 15.02.2002
(51) Int. Cl.: C07K 14/47, C12N 15/63, C12N 15/12, A61K 38/17

(54) **Porcine complement regulator factor H and its use**

(71) Applicant: HANS-KNÖLL-INSTITUT FÜR NATURSTOFF-FORSCHUNG e.V., 07745 Jena (DE); Zipfel, Peter, F., Prof. Dr., 07745 Jena (DE); Hegasy, Guido, Dr., 20253 Hamburg (DE)
(72) Inventor: Hegasy, Guido, Dr., 20253 Hamburg (DE); Zipfel, Peter F, Prof. Dr., 07745 Jena (DE)

(57) **Abstract**

Porcine complement regulator factor H (pFH) is capable of interacting with human molecules, e.g. human complement components and heparin, and showing enzymatic activity as a cofactor for human complement. In particular, but not exclusively, pFH exhibits regulatory functions for the complement system in different species. Accordingly, the invention relates to porcine complement regulator factor H (pFH) and to biologically and / or enzymatically active fragments, variants and mutants thereof, and to the use of such pFH as well as fragments, variants and mutants thereof to control complement activation.

## Description

### Field of the Invention

The present invention concerns a porcine protein termed pFH and its encoding DNA. The protein pFH is capable of interacting with human molecules, e.g. human complement components and heparin, and showing enzymatic activity as a cofactor for human complement. In particular, but not exclusively, pFH exhibits regulatory functions for the complement system in different species. Further interaction of pFH with natural or artificial surfaces and with other molecules are included.

### Background of the Invention

The complement system provides an efficient way for the recognition and destruction of structures and surfaces exposed to blood and discriminated as foreign by the host organism. Three major pathways of activation are described ¹. The classical pathway (CP) is activated by immunglobulins upon binding to foreign epitopes. The lectin pathway is activated by carbohydrate structures and the alternative pathway (AP) is independent of antibodies and occurs constantly in human plasma by spontaneous hydrolysis of C3 ². Subsequent generation of C3 convertase results in a feedback amplification loop that allows formation and deposition of a large number of C3b molecules on unprotected surfaces. The results are the formation of the membrane attack complex, release of toxic complement products and destruction of the unprotected surface. This tick-over activation by the AP ensures a mechanism of constantly checking plasma exposed surfaces for their activation potency ³. Differentiation between activator and non-activator surfaces is ensured by regulators ⁴. As a crucial human complement regulator factor H (FH) determines the fate of newly formed C3b and controls formation and stability of C3 convertases both in fluid phase and on surfaces ⁵⁻⁷. Beside membrane-bound regulators, the affinity of a surface for FH regulates its activation potency. Binding to heparin is a a widely used in-vitro model for the demonstration of this FH / surface interaction ⁴.
Human FH is a single-chain glycoprotein with a molecular mass of 150 kDa and has a physiological plasma concentration of approx. 500 µg/ml. FH is mainly synthesized in the liver and secreted to the blood. The mature protein is composed of 20 repetetive elements termed short consensus repeat (SCR) ⁸. Some functional domains of human FH have been mapped to several SCRs. Reduction, absence or mutations of FH can lead to uncontrolled complement activation. Severe diseases as kidney damage (glomerulonephritis of different kinds), hemolytic uremic syndrome (HUS) and / or relapsing infections ⁹ are the result. FH belongs to the FH gene family that includes the factor H like proteinl (FHL-1) and factor H related proteins 1 to 5 (FHR-1 to FHR-5). FHL-1 is composed of the first seven SCRs of FH and 4 unique amino acids. FHL-1 shares the complement regulatory functions with factor H and interacts with heparin ^{10;11}. Sequence analogues of human FH have been described so far in rat ¹², but only partially in mouse ¹³ and very truncated in bovine ¹⁴. Cloning of the pig analogue has failed totally to date. The rat analogue of FH has been expressed recombinantly in a truncated form and the native protein was purified from rat blood. Enzymatic activity studies were undertaken with the native and the truncated protein. A generally accepted in-vitro model for the demonstration of regulatory activity of FH is the co-factor assay. In this assay FH is incubated with Factor I and C3b. If FH displays co-factor activity C3b is degraded in the presence of FI and FH. In the case of rat FH cofactor activity could only be demonstrated if incubated with rat C3b and rat FI, but not with human C3b and human FI¹². Thus rat FH was shown not to be interchangeable with human FH. This finding is in good agreement with the fact that complement regulation in general is considered to be species restricted. Complement regulatory proteins fulfill their function basically only when interacting with complement from the same species ¹⁵. This species restriction is of importance in different clinical situations. Since complement plays an important role in the cross-species transplantation of porcine livers into humans, successfull efforts have been undertaken to generate pigs transgenic for two membrane bound complement regulators, the human DAF and the human MCP protein ¹⁶. The effort that has been undertaken to generate these transgenic pigs stresses the species restriction of complement regulation. The following invention provides now for the first time the DNA and the protein sequence of the porcine protein termed pFH that is able to cross the species restriction border and to regulate human complement.
The present invention relates to the porcine protein termed pFH and its encoding DNA. In the present invention it is shown for the first time that the porcine protein pFH is able to interact with human C3b and human FI. Surprisingly, porcine pFH shows regulatory and enzymatic activity in a human protein context. In contrast to other complement regulatory proteins pFH displays activity outside its species of origin and is capable to act as a cofactor for human complement proteins. This activity is shown to be of an unexpected intensity making it an equivalent to human FH in a qualitative and quantitative way. We are showing that the molecule interacts with human C3b (hC3b) and human FI (hFI) and shows biological activity in a human protein context. It can be used as a cofactor for the hFI-mediated cleavage of hC3b. Furthermore the protein pFH is capable of interacting with heparin as a widely used in-vitro model for surface interaction.

### Description of the Invention

The invention relates to a porcine complement regulator factor H (pFH) protein selected from the group consisting of mature pFH, a biologically and / or enzymatically active fragment of said mature pFH, and a biologically and / or enzymatically active variant or mutant of said mature pFH or fragment thereof. In an especially preferred embodiment the invention concerns substantially pure pFH having the amino acid sequence depicted in SEQ ID NO 2.
In a further embodiment the invention relates a nucleic acid selected from the group consisting of a nucleic acid coding for a pFH protein as defined above and the corresponding complementary nucleic acid. A preferred nucleic acid of the invention is the DNA having the nucleotide sequence depicted in SEQ ID NO 1.

Production of pFH can be achieved either by recombinant expression or by purification from porcine blood. The protein can be expressed recombinantly using the DNA sequence of this invention as shown in Example 4. Recombinant expression is preferably achieved in eucaryotic expression systems. As shown in Example 4 expression in the baculovirus system results in high yield of functionally active pFH. Other eucaryotic expression systems suitable for the expression of pFH are known per se. pFH can be expressed as a whole protein (identical to pFH SCR 1-20) or only functional parts of it. Functional domains are located for example on SCR 1-4, SCR 5-7, SCR1-7 and SCR 15-20 as shown in Examples 4 - 6. Recombinant expression of functional fragments of pFH may be desirable to uncouple the different functions of the protein from each other, for reasons of separation of functional domains, molecule size, antigenity or for other reasons. Functional domains remain functionally active if expressed separately as shown in Examples 4 - 6. Additional sequences coding for sites from other proteins can be added to the sequence of pFH without loss of activity of the pFH domains. Two sequences were added to the fragments in Example 3: A human secretion sequence for the release of pFH from cells of the baculovirus system and a 8xHistidine-Tag for purification with the metal-chelate system. Functional activity of the expressed fragments remained intact. A further embodiment of the invention is a vector comprising a nucleic acid sequence coding for a pFH protein as defined above.

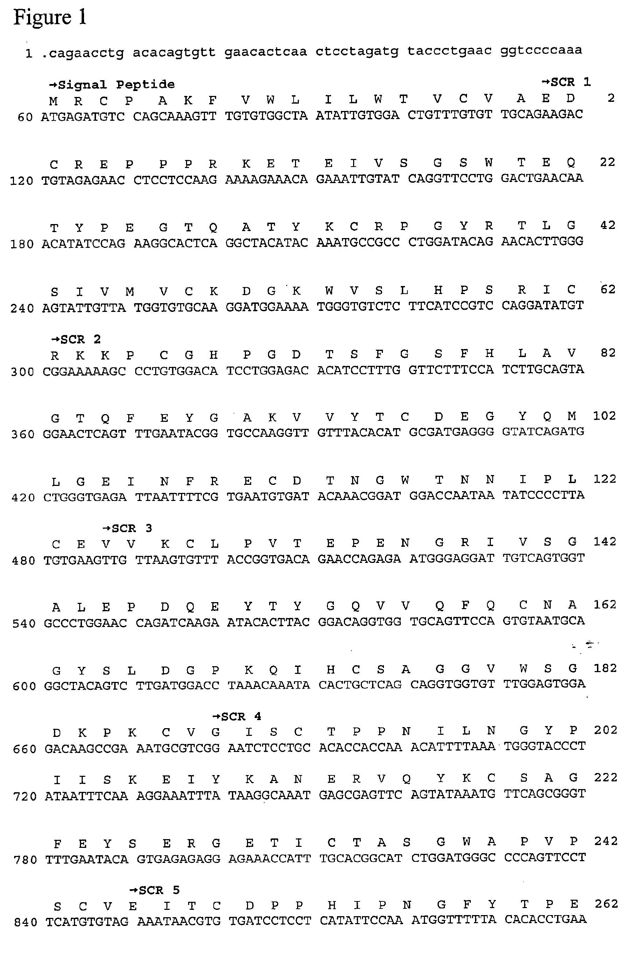

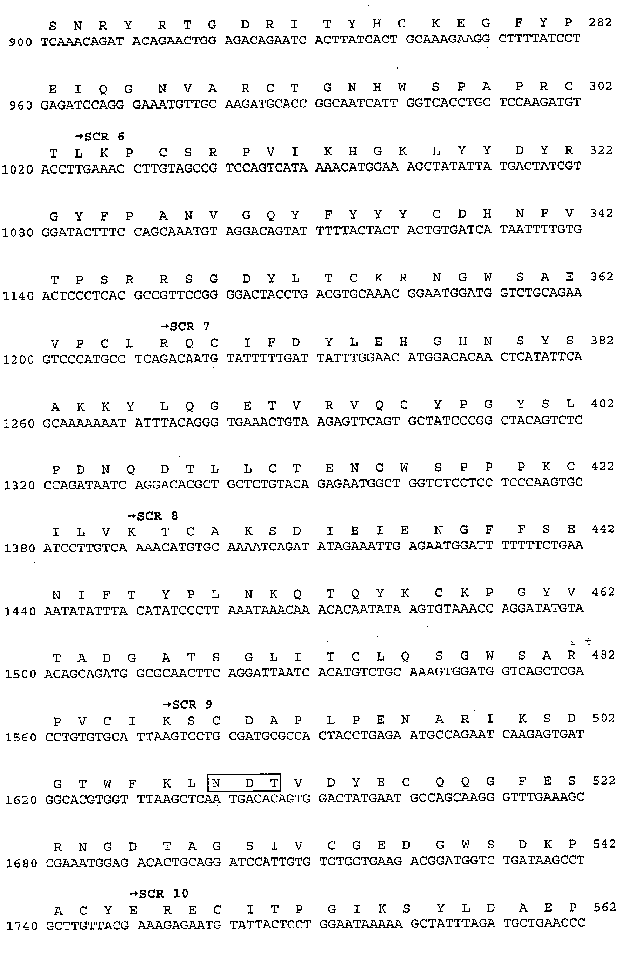

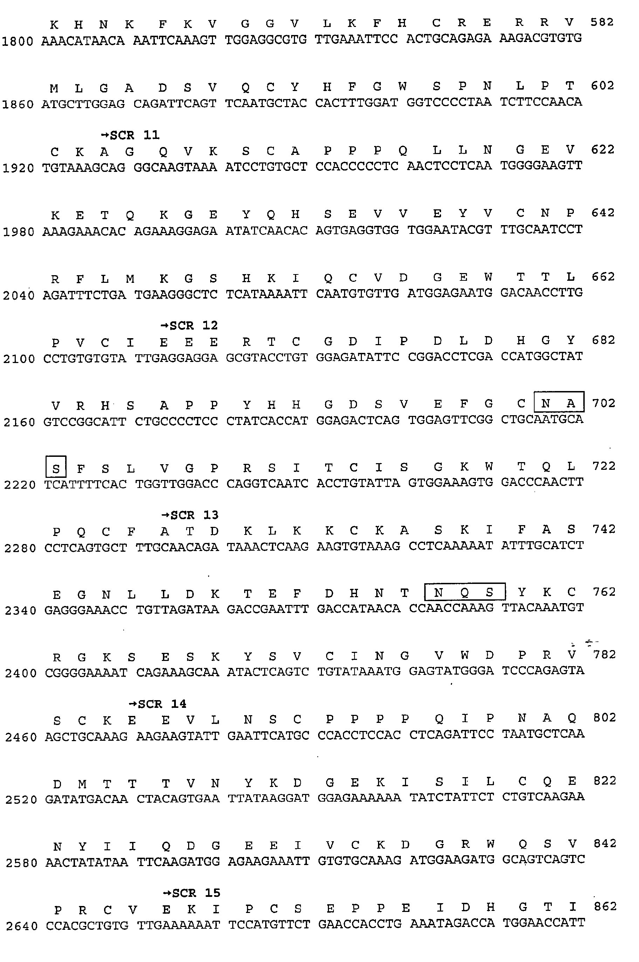

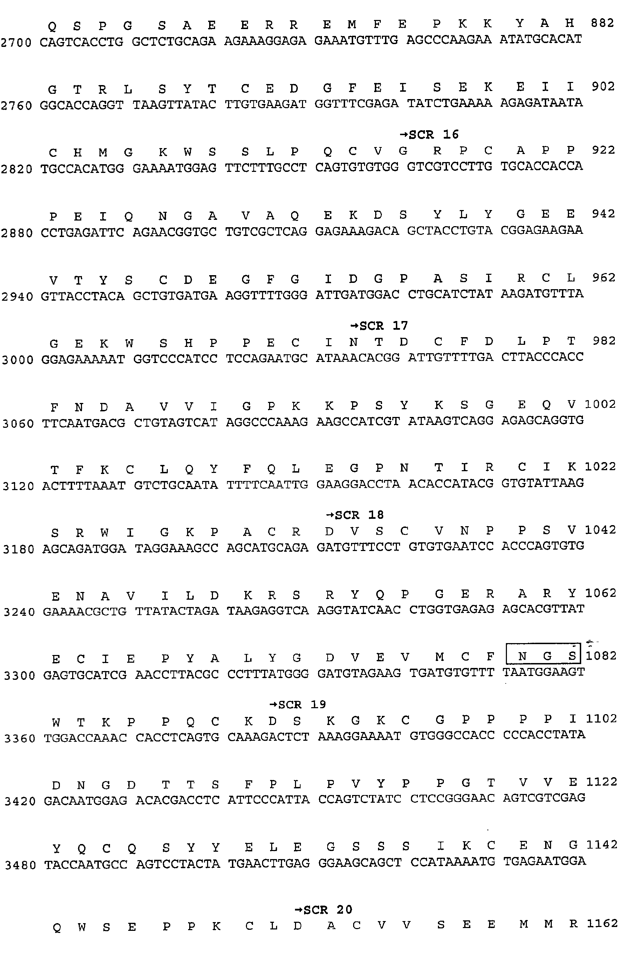

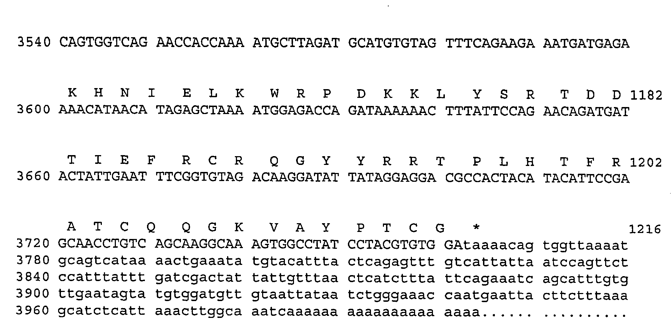

Plasmid pigFH containing the complete pFH DNA as depicted in Figure 1 and SEQ ID NO 1 has been deposited on 22.01.2002 under Accession No. DSM 14763 with the Deutsche Sammlung von Mikroorganismen und Zellkulturen GmbH (DSMZ), Mascheroder Weg 1b, D-38124 Braunschweig. The deposition has been made under the Budapest Treaty on the International Recognition of the Deposit of Microorganisms for the Purposes of Patent Proecedure.

Alternatively, pFH can be purified from porcine blood that is obtained in abundance as a worthless byproduct of regular meat production and is thus a cheap source material for purification of pFH. Purification methods for complement proteins are known per se,^{17;18} e.g. affinity chromatography or gel filtration. Furthermore purification can be achieved by heparin affinity chromatography as shown in Example 4 or by standard immunoaffinity chromatography with the antibody anti-pFH used in Examples 4 and 6. Standard purification methods for FH are described ¹⁹. Truncation from the purified protein pFH can be achieved by standard protein digestion methods. Protein digestion methods are known per se and carried out by enzymatic or chemical treatment. Enzymatic treatments are for example trypsin or protease V8 digestion. Chemical treatment is for example hydrochloric acid digestion.

Alternatively pFH expression levels can be modified within the porcine liver itself. Enhanced endogenous production of pFH by the porcine liver can be achieved by modification of the pFH DNA. Different ways of upregulation of pFH expression can be applied: by genetic modification of expression regulatory elements, e.g. the promoter sequence, and by the use of exogenic or endogenic molecules that enhance pFH expression, or other procedures. Modification of the promoter sequence can allow to regulate the expression level of the endogenous production of the pFH protein. Standard techniques for promoter modifications are known per se. The pFH sequence can be used as the basis for the genetic modification of the found sequence in order to enhance the expression level of pFH. The sequence can be used as the basis for any type of modification of the genomic wild-type sequence and its alleles. Stimulation of the pFH promoter can be achieved by the use of endogenic substances, e.g. cytokines from the groups of tumor necrosis factors, interleukines or interferones or other, and exogenic substances, e.g. from the group of cortisol and its pharmacological derivatives as it has been demonstrated for human FH ^{20;21}.

A further embodiment of the invention relates to the use of a nucleic acid coding for a pFH protein as defined above for the preparation of a cell, tissue or animal wherein said cell, tissue or animal expresses or is capable of being caused to express increased amounts of a pFH protein as defined above.

The invention relates to the use of a pFH protein selected from the group consisting of mature pFH, a biologically and / or enzymatically active fragment of said mature pFH, and a biologically and / or enzymatically active variant or mutant of said mature pFH or fragment thereof, as an agent to control complement activation in animal species, especially mammals, most preferably humans. Especially, the invention relates to a pFH protein as defined above for use in a method for the prophylactical or therapeutical treatment of the human or animal body. Examples for preferred prophylactical or therapeutical treatments in humans are the control of complement activation in the course of non-human animal(such as pig)-to-human liver xenotransplantation and the control of complement activation in cases of FH deficiencies. The control of complement activation may also be performed in vitro or ex vivo. For example, whenever blood (in vitro or ex vivo) comes in contact with artificial surfaces a pFH protein as defined above can be used to control complement activation by coating of said surfaces.

The present invention concerns the application of pFH or parts thereof in any way described above. The prepared proteins of pFH can then be used in all clinical situations, where complement activation occurs, but is harmful. Clinical situations can be inborn deficiency or inactivity of human FH. Other clinical situations where harmful complement activation occurs are phases of organ ischemia for different reasons. Ischemia occurs while an organ is withdrawed from regular blood supply. This situation occurs e.g. during interventional or thrombotic occlusion of blood vessels as described below. Furthermore it relates to other functions of the protein or its fragments, e.g. the ability of surface and heparin interaction or binding to human proteins with or without enzymatic activity. More detailed, the invention provides the porcine molecule pFH, that can be administered as a drug to control complement activation in humans. Preparations of pFH can be administered intravenously or intramuscularly. Application can be to achieve high pFH plasma level if human FH is normal or subnormal. To compensate subnormal hFH plasma level in patients pFH can be administred by repeated injections or continuous application in a range of 0.5 mg/L to 1000 mg/L diluted in physiological NaCl solution or Ringer-Lactat solution, both frequently used in infusion therapy. In ischemia therapy pFH can be administred in a concentration range of 1 g/L to 10 g/L together with common thrombolytic substances, e.g. heparin or tissue plasmin activator to prevent complement activation after re-opening a closed blood vessel ²². pFH can be given constantly intravenous or via a catheter placed in the vicinity of the thrombembolus as it is common routine in infarction therapy. To control complement activation in surgical organ taking pFH can be administred to the organ itself or systemically to the body. In organ transplantation pFH can be perfused into the vessel of the transplant organ in a range of 0.5 mg/L to 1000 mg/L together with common transplantation perfusion solutions, e.g. EuroCollins, HTK- or Bretschneider-solution ²³. The invention relates, accordingly, also to a pharmaceutical composition comprising a pFH protein as defined above.

The protein pFH or its fragments can be used to coat biomaterial surfaces that come in contact with human blood or plasma and thus prevent harmful complement activation that occurs otherwise. Coupling of pFH to artificial surfaces can be achieved by standard protein coupling reactions, e.g. amine-coupling methods. Coupling to surfaces in order to prevent complement activation has been demonstrated for human FH ²⁴. Other coupling methods are known per se, e.g. photoreactive coupling. Surfaces can be modified to allow easier coupling of pFH by its natural domains or by generation of chimeric proteins with pFH domains. pFH coated surfaces can be used wherever blood or plasma comes in contact with artificial surfaces inside or outside the body.

### Fields of Application of pFH

The complement regulatory and interacting capabilities of pFH and its fragments can be used in different physiological, pathological or other situations, some of them exemplarily described in the following.

### Application of pFH to control Complement Activation in Pig-to-Human Liver Xenotransplantation

Liver transplantation has become a routinely practiced medical intervention. Due to the shortage of available human organs the pig has been chosen as a source for xenotransplantation organs. The first major hurdle in carrying out a cross-species xenotransplantation is the occurance of Hyperacute Rejection (HAR) triggered by complement activation. Activation of the complement system via both pathways has been shown to be of relevance for HAR: The CP is activated by preformed host antibodies against structures on the donor organ vessel endothelium. Carbohydrate groups (mainly the alpha-galactosyl epitope ²⁵) that only appears in animals but not in humans are the most immunogenic structures in this activation pathway. Activation of the AP has been shown to occur in the pig-to-primate transplantation although the activation mechanism remains unclear ^{26;27}. Furthermore the AP plays a central role in amplification of the complement cascade once it has been initiated by the CP. The second rejection phase termed Acute Vascular Rejection (AVR) has not been shown to be excluded from complement activation. To date experimental horizons do not consider rejection phenomenons beyond these two phases, but relevance of the complement system might be given. Control over human complement activation has been tried to achieve in several ways. The following three are the described ways to date of controlling complement activation in xenotransplantation, reviewed in ²⁸. They are reported together with their known disadvantages:
1. Administration of pharmacological drugs to regulate complement activation in fluid-phase, e.g. Cobra Venom Factor (CVF) and soluble complement receptor 1 (sCR1). Control over complement activation was shown for these drugs, and survival of the organ acceptor animal was indeed prolonged. Still median survival lies around hours up to several days with only very few publications claiming weeks of survival. Longterm administration of these drugs is limited by their toxicity and the fact that the complement system has to be kept intact in the long run. Otherwise a functional complement deficiency was created leading to known diseases as MPGN II, HUS and relapsing episodes of infection as shown in animals and humans ²⁹. Pharmacological complement inhibition must therefore be only a temporary condition.
2. Removal of animal carbohydrate epitopes on the donor organ endothelium (alpha-galactosyl epitope) by genetic modification of the enzyme responsible for generation of this sugar epitope. Positive effects have been shown for these treatments, but epitopes were always removed only in part and complete abrogation of the carbohydrates has never been achieved. Furthermore the AP activation remains unrestricted by this approach since it is not dependent on antibodies.
3. Donor pig transgenity for human surface-bound regulators. As it has been shown that complement regulation can be species restricted, pigs have been made transgenic for the human surface-bound complement regulators DAF and MCP. This strategy appears to be effective, but regulation of the complement attack takes place only on the cell surface stage, while the fluid phase generation remains active. Furthermore it is difficult to insert and regulate complete human genes in animals while the use of endogenous protein-coding sequences is more effective, easy and elegant, provided that it can regulate human complement.

The porcine protein pFH offers now for the first time the possibility to use endogenous porcine regulators to prevent the activation of human complement. The regulation concerns the CP, the LP and the AP and takes place in the fluid phase as well as in contact with surfaces and thus at the first stage of activation. Prerequisite is the ability of the protein to cross the species restriction border and interact with human complement proteins. The effort that has been undertaken to make pigs transgenic for human surface-bound complement regulators ¹⁶ shows the general species restricition of complement regulation. Unexpectedly the protein pFH displays several activities known to be crucial for regulation of human complement activation: 1st Affinity for heparin, 2nd Affinity for human C3b and human FI, 3rd Cofactor activity in the cleavage of human C3b by human FI.
The porcine molecule of this invention displays regulatory capabilities for the human complement proteins. Interaction of pFH with heparin is demonstrated by binding studies in Example 3. Affinity and enzymatic activity is demonstrated by cofactor cleavage assays with human C3b and human FI in Example 4 and 5. pFH is shown to act as a cofactor for human FI in the cleavage of human C3b.
The invention is based on the concept of manipulating the found endogenous porcine fluid-phase regulator for controlling complement activation in xenotransplantation. Hyperexpression of the found molecule can regulate the human complement activation and thus influence the outcome of HAR and later phases of organ rejection.

### Application of pFH as an Agent to control Complement Activation in Humans

Deficiency or mutations of Factor H have been described in several cases in humans ²⁹. Three different diseases are frequently reported to be associated with FH deficiency, reviewed in ⁹: 1. Relapsing episodes of Hemolytic Uremic Syndrome (HUS), 2. Membranoproliferative Glomerulonephritis Type II (MPGN II), 3. Increased susceptability to infections. Treatment of these patients with human plasma to restore normal FH plasma levels can be of benefit ³⁰. This therapy can restore normal and functional FH plasma levels but due to the half-live of FH in plasma the improvement will be only temporarily. The known risks of human plasma transfusion (e.g. viral infections, incompatability reactions) combined with questions of expense and efficacy limit the possible treatment to acute situations and experimental conditions.
Furthermore harmful complement activation has also been demonstrated in pathological situations where FH is present in human plasma but complement is still excessively activated. Pathological complement attack occurs e.g. while an organ has been withdrawn from normal blood supply and is reperfused later. It has been shown that not ischemia of an organ is the damaging event but that reperfusion starts the pathological chain reaction leading to the final necrosis and organ destruction ^{31;32}. This reperfusion injury is triggered by AP complement activation and is considered the main factor in destruction of the ischemic organ. Reperfusion Injury occures e.g. during: 1st Myocardial infarction; 2nd Brain infarction; 3rd other organ infarction, e.g thrombosis and embolism; 4th surgical blood vessel clamping; 5th surgical blood vessel ligation for any reason, e.g. for organ taking.
The invention is based on the concept of regulating the human complement system using the porcine molecule pFH. Since pFH is shown to cross the species restriction border and act as a cofactor for hFI in the cleavage of hC3b, it can be used as an agent to control harmful complement activation in humans. Thus preparations of pFH can be administered whenever human complement activation is harmful and has to be prevented.

### Application of pFH for Surface Coating to control Complement Activation on blood exposed artificial Surfaces

In many types of medical devices blood comes in contact with artificial surfaces. In extracorporal devices like hemodialysers, oxygenators or plasmapheresis equipment, and intracorporal devices like catheters, stents, shunts, vascular grafts or heart aids, blood and technical surfaces come in contact triggering the AP of the complement system. This activation leads to the production of toxic products and activates platlets and leukocytes. Among others an inflammatory reaction of the patients immune system is the result of this process and can result in reactions ranging from mild anaphylactic reaction to shock and death ²⁴. The AP of complement activation has been found to be the essential activation pathway in this interaction and clinical symptoms are mediated by the release of activated complement components. Control of the AP activation has been tried to achieve by covering exposed surfaces with heparin but results are not optimal.
The invention is based on the concept of covering all surfaces that come into contact with blood with the protein pFH or ist fragments in order to prevent complement activation.

The following examples serve to exemplify the invention but should not be construed as a limitation thereof.

### Experimental Part

### Material used in the Examples

### Molecular biology

All general reagents were obtained from Sigma (Taufkirchen, Germany) and all labware were obtained from Greiner (Solingen, Germany) unless otherwise stated. TRIzol Reagent was obtained from Life Technologies (Karlsruhe, Germany). All restriction enzymes, Taq DNA polymerase and [α-³²P]dATP Redivue were obtained from Amersham Pharmacia Biotech (Freiburg, Germany). T4 DNA-Ligase was obtained from Roche (Mannheim, Germany). Nytran nylonmembranes and Protran nitrocellulose membranes were obtained from Schleicher&Schuell (Dassel, Germany). Poly-ATtract mRNA isolation system and Prime-a-Gene labeling system were obtained from Promega (Mannheim, Germany). From Qiagen (Hilden, Germany) came QIAprep miniprep spincolumns, QIAquick gel extraction kit, QIAquick PCR purification kit and Ni²⁺-NTA Agarose resin. Microspin S300 columns were obtained from Bio-Rad (Muenchen, Germany). ZAP-cDNA Sythesis Kit and ZAP-cDNA GigapackIII Gold Cloning Kit were obtained from Stratagene (Amsterdam, Netherlands). Dialysis tubing Spectrapor6 MWCO 1000 was obtained from Serva (Heidelberg, Germany). Ultrafree-15 centrifugal filter device Biomax-5k were obtained from Millipore (Eschborn, Germany). BCA protein assay reagents were obtained from Pierce (Rockford, USA). BaculoGold Baculovirus DNA was obtained from BD Pharmingen (San Diego, CA, USA). BigDye Terminator Kit for sequencing reaction was obtained from Applied Biosystems (Langen, Germany).

### Tissues, cells and media

Fresh pig liver tissue was obtained from Schlachterei Ernst (Hamburg, Germany). Slaughter of the animal was solely for meat production. The tissue source was a female pig (sus scrofa), 6 month of age and breeding type Deutsches Mastschwein. Liver tissue lumps were excised after regular dissection of the desribed animal and immediately snap-frozen in liquid nitrogen. Spodoptera frugiperda cells (SF9) were grown at 28°C in monolayer culture in X-press medium (BioWhittaker, Verviers, Belgium) containing streptomycin (100µg/ml), penicillin (100U/ml), amphotericin B (250ng/ml) and 4% fetal calf serum, all purchased from Life Technologies (Karlsruhe, Germany). For protein expression FCS was left out of the media.

### Proteins and antibodies

Anti-pFH(rabbit) antibody was obtained from Dr.Kolbjørn Høgåsen (Lillehammer, Norway). HRP-conjugated secondary antibody α-rabbitIgG(goat) was obtained from Dako (Glostrup, Denmark). Human C3b, human Factor H and human Factor I were obtained from Calbiochem (Schwabach, Germany). Human FHL-1 was expressed recombinantly in the baculovirus system. HiTrap Heparin Columns were obtained from Amersham Pharmacia Biotech (Freiburg, Germany).

### Technical equipment

Stratalinker 1800 was obtained from Stratagene (Amsterdam, Netherlands). Sequencing was performed using an ABI PRISM 377 Sequencer from Applied Biosystems (Langen, Germany). PCR was performed using a GeneAmp 9700 thermal cycler from Applied Biosystems. ÄKTAprime instrument was obtained from Amersham Pharmacia Biotech (Freiburg, Germany).

### Example 1: Generation of the Pig Liver cDNA Library

A pig liver cDNA library was generated since the only commercially available library (Clonetech porcine liver cDNA library, PL 1011b) could not reveal any clones neither by probe or antibody screening nor by PCR amplification. At the time of research for the present invention, this was the only commercially available porcine liver cDNA library worldwide. Thus pFH could only be found by construction of a pig liver cDNA library and not by using a commercially available one.
Approximately 0.9 g of frozen pig liver tissue were ground to powder with mortar and pestle under constant addition of liquid nitrogen. The powder was then transferred to a Falcon containing 9 ml of prewarmed Trizol-Reagent. The solution was homogenized by vigorous vortexing and aliquots of 1.5 ml were transferred to fresh tubes. Each tube was processed seperately, starting with the addition of 300 µl of Chloroform. RNA pellets were redissolved in ddH₂O and pooled. OD₂₆₀-quantification resulted in 1,87 mg total RNA in with an A²⁶⁰_{/280} Ratio of 1.63. 1 mg total RNA was used as a starting material for poly(A)mRNA isolation with the PolyATtract system. 12.5 µg poly(A)mRNA with an A²⁶⁰_{/280}-Ratio of 1.66 were obtained.

The library was generated using the Stratagene ZAP-cDNA Sythesis Kit and the GigapackIII Gold Cloning Kit. 5 µg mRNA dissolved in 20 µl ddH₂O were used for First- and Second-Strand cDNA Synthesis. As a synthesis control [α-³²P]dATP was incorporated in the reaction. Ligation of the EcoRI Adapters was carried out at 5 °C for 2 days. Size fractionation was achieved by separation on a drip column. An 1.4 ml insulin syringe was used as a column filled with CL2B-Sepharose and loaded with the digested cDNA. 7 Fractions were collected and analysed by 5 % nondenaturing acrylamide gel electrophoresis followed by exposition to x-ray film overnight. The third fraction contained cDNA ranging from approximately 0.6 to 4 kb and was selected for further processing. 1 µl was ligated into Uni-ZAP XR Vector for 2 days at 4°C and then packed with the GigapackIII Gold Kit. The unamplified library was then plated out for titering. Approximately 15.000 plaques formed on a 150 mm plate from 1 µl of the packaging reaction. 18 plaques were randomly picked, eluted and the sizes of the inserts analysed by PCR. The generated unamplified library was analysed by insert size determination from 18 individual clones picked seperatly from the first plating. Size range of the inserts as determined by PCR was between 0.8 kb and 3.9 kb with an average insert size of 1.6 kb. 1µl unamplified library plated out on a 150mm plate resulted in approximately 15.000 pfu. Only one blue plaque was observed with blue/white selection on this plate. No amplification was carried out before the subsequent screening.

### Example 2: Screening of the Pig Liver cDNA Library for pFH

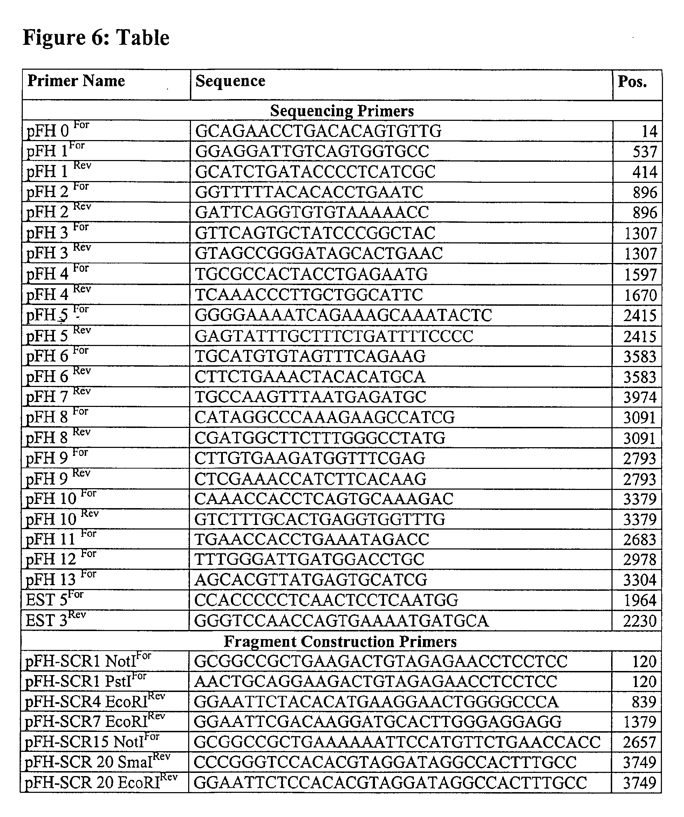

Database alignments of the known human FH sequence and a pig small intestine EST database was carried out ³³. One EST clone among 893 clones showed homology to human FH and was chosen for primer design. The primers termed EST 5^{For} and EST 3^{Rev} generated a 291 bp fragment from a 1:10 dilution of the third cDNA library fraction. Primer sequences are shown in Figure 6 (Table). PCR-conditions were: 95°C 5 min, 30 amplification cycles of 95°C 45sec/ 56°C 45sec/ 72°C lmin, followed by 72°C 10min. The PCR-product was resolved on an 1% agarose gel. The fragment was cut out and eluted from the slice using QIAquick gel extraction columns. 4µl elute was used as a template for Prime-a-Gene labeling reaction. [α-³²P]dATP was used as the labeling radionucleotide. Unincorporated nucleotides were removed using Microspin S300 columns.
The generated pig liver cDNA library was plated out at ∼30.000 pfu per NZY-plate on 11 plates with a lawn of XL1-Blue *E.coli*, allowing a screen of 330.000 independent unamplified clones. The plates were grown overnight at room temperature followed by growth at 37°C until near confluency was reached. Duplicate lifts were taken using Nytran filter membranes. Denaturation was performed by placing the membranes on Whatman paper saturated with 1.5 M NaCl/0.5M NaOH, neutralized in 1.5M NaCl/0.5M Tris (pH7.5) and washed in 0.2M Tris (pH 7.5)/2x SSC. DNA was then UV-crosslinked with 1200 kJoule using the Stratalinker 1800. Membranes were prehybridized in Church-Mix (0.5M Na₂HPO₄, 7% SDS, 1mM EDTA, pH7.2) for 1 hour at 50°C before addition of the denatured radiolabeled probe and then incubated overnight. After washing the filters in Church-Wash (40mM Na₂HPO₄, 1%SDS, pH7.2) at 50°C twice for 15 min they were sealed and exposed to x-ray film for 2 days at -70°C. Positive Plaques were picked and eluted in 250µl SM buffer. Screening of the library resulted in 48 positive clones on both replicates of plaque lifts. All clones were picked, eluted and analysed for their insert size. Inserts were controlled by PCR using EST 5^{For} and EST 3^{Rev} primers and standard plasmid-specific primers M13^{For} and M13^{Rev}. Cycling conditions were: 95°C 5min denaturation, 30 amplification cycles of 95°C 45sec/ 56°C 45sec/ 72°C 2 min, followed by 72°C 10min. The 5' and 3' cDNA ends of the inserts in relation to the EST were analyzed by this approach. Five clones producing the longest fragment for 5' PCR amplification were picked and rescreened as described above. The clone with the longest insert in the second screen was chosen for further processing. The cDNA was recovered from the isolated phage by In Vivo Excision using the Stratagene ExAsisst helper phage and the SOLR strain. Individual bacterial colonies containing the phagemid were grown overnight in LB-Ampicillin and the plasmid DNA was purified using Qiagen Miniprep spincolumns.
The cDNA insert of said plasmid named pigFH (DSM 14763) was sequenced in both directions using the primers shown in Figure 6 (Table). The nucleotide sequence and the derived amino acid sequence are shown in Figure 1. Comparison of the nucleotide sequence and the deduced amino acid sequence showed homology with known Factor H sequences. Thus the cDNA insert was designated pFH (GenBank Accession Nr. AJ278470, confidential until 15.06.2002).
The clone sequence starts with 59 bases 5' untranslated region. A signal peptide sequence of 18 amino acids directs the protein to the secretory pathway. The mature protein consists of 1216 amino acids with 20 SCRs and a molecular weight of approx. 136 kDa. The 3'UTR has 222 bases followed by the polyA tail. pFH has 4 potential glycosylation sites, one of which is identical to the human site in SCR 9. The EST sequence is only 90% homologue to the found sequence. This low homology is mostly due to sequencing errors by missing bases in the published EST sequence. Homology along the whole sequence was 62% for pFH and human FH, 58% for pFH and mouse FH and 60% for pFH and rat FH.

### Example 3: Construction of four pFH Fragments

Four fragments were generated consisting of pFH SCR1-4, SCR1-7, SCR15-20 and SCR 1-20. The fragment pFH SCR 1-20 is analogue with the complete pFH protein as it is found in pig plasma. The isolated pFH plasmid served as a template for PCR reactions. Primers used for construction of pFH fragments are listed in Figure 6 (Table) and SEQ ID NOs 3-34. Fragment pFH SCR 1-4 was amplified using primers pFH SCR1-PstI^{For} and pFH SCR4-EcoRI^{Rev} containing the appropriate restriction site indicated by the name. Fragment pFH SCR1-7 was amplified using primers pFH SCR1-NotI^{For} and pFH SCR7-EcoRI^{Rev}. pFH SCR15-20 was amplified using pFH SCR15-NotI^{For} and pFH SCR20-EcoRI^{Rev}. Fragment pFH SCR 1-20 was amplified using primers pFH SCR1 NOtI^{For} and pFH SCR 20 SmaI^{Rev}. All four fragments were amplified according to the following PCR protocol: 95°C 5min denaturation, 35 amplification cycles of 95°C 45sec/ 58°C 45sec/72°C lmin30sec, followed by 72°C 10 min. The fragments were gel-purified and double-digested with the appropriate enzymes (NotI, SmaI, EcoRI and/or PstI). The digestion reaction was purified using Qiagen PCR purification columns and then ligated into the predigested baculovirus expression vector pBSV8-His. This expression vector generates a chimeric protein that carries an additional secretion sequence for purification from cell culture medium and a 8xHis-Tag for easy metal-chelate purification ³⁴. To confirm the correct sequence of the inserted DNA all plasmids were transformed into competent DH5α cells, purified and sequenced.

### Example 4: Recombinant Expression of four pFH Fragments.

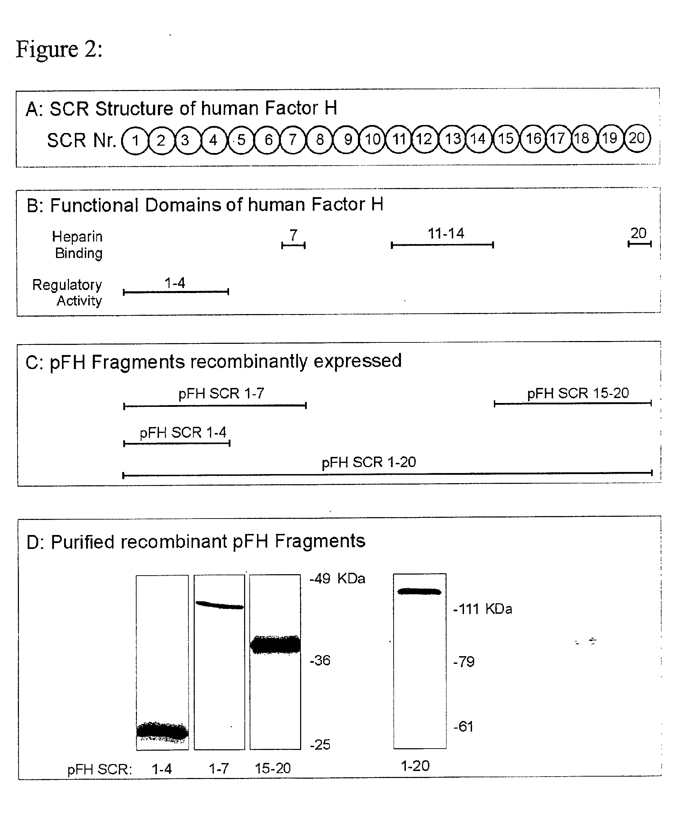

Sf9 cells (3x10⁶/ml) were grown in FCS-supplemented medium and infected in protein-free X-press Medium with recombinant virus using. The culture medium was harvested on day 10 post infectionem and directly used for metal-chelate purification. 40ml culture supernatant were incubated overnight with 5ml nickel-NTA agarose. Every step was carried out at 4°C and the resin was precipitated every time by centrifugation (450 x g, 4°C, 10 min).The following washing steps were carried out: Twice with 40ml buffer (0.5M NaCl, 20mM Tris-HCl, pH 7.9) containing 5mM imidazole, once with buffer containing 30mM imidazole and once with buffer containing 50mM imidazole. Proteins were eluted twice in buffer containing 1M imidazole. 80 ml elute was dialysed twice against 4 liters of 1xVBS buffer and then concentrated with an Ultrafree-15 centrifugal filter device. Purity and integrity of the proteins were analyzed by SDS-PAGE and subsequent silver staining. Protein quantity was measured using the standard BCA protein assay. SDS-PAGE was performed using 10% or 12% separating minigels under non-reducing conditions.
The known structure and function of human FH and the expressed fragments of pFH are shown in Figure 2. Organization of human FH in SCRs is shown in Panel A. Functional domains of human FH that are known to date are shown in Panel B. Human FH SCR 1-4 fragment carries the regulatory activities (cofactor activity and decay-accelerating activity). Fragment hFH SCR1-7 has an additional heparin binding site in SCR 7. Human FH SCR15-20 carries a heparin binding site but no known regulatory activity. The structures of the expressed fragments representing pFH SCR1-4, SCR1-7, SCR15-20 and SCR 1-20 (complete pFH) are shown in Panel C. Fragments were chosen according to putative functional domains known in human FH. Silver stain detection of expressed pFH fragments are shown in Panel D. Abundant amounts of protein of all four fragments could be detected in purified SF9 cellculture supernatant as shown.

### Example 5: Binding of pFH Fragments to Heparin.

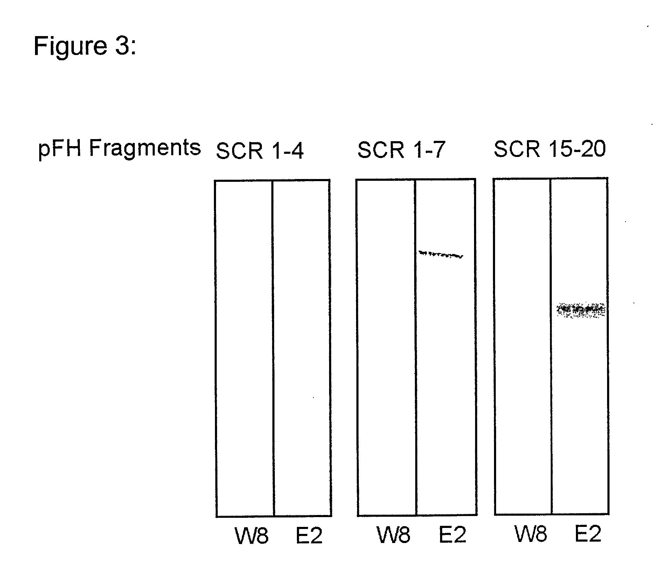

10 ml of cell culture supernatant containing the recombinant fragment pFH SCR1-4, pFH SCR1-7 or pFH SCR15-20 diluted in equilibration buffer (1/3x PBS, 50 mM NaCl), were applied on a 1 ml sepharose column coupled with 10 mg heparin. Samples were passed over the column at a flow rate of and 1 ml/min at room temperature and the fall-through was collected by the ÄKTAprime instrument. Columns were washed 8 times with 20 column volumes of equilibration buffer. Possible bound proteins were eluted by a NaCl gradient from 50 mM to 500 mM NaCl in 8 elution steps. Washing and elution fractions were subjected to SDS-PAGE and subsequent Western-blotting. Blotting membranes were blocked with 5% dried milk in PBS for 30 min and incubated overnight with anti-pigFH(rabbit) diluted 1:400 in PBS with 2% dried milk. Secondary antibody was a HRP-conjugated anti-rabbit-IgG(goat) antibody diluted 1:1000 in PBS 2% dried milk and incubated for 3 hours. The membranes were developed in chromogenic solution (45ml PBS, 5ml Methanol, 15mg 4-chloro-1-naphtol and 50µl H₂O₂). Results are shown in Figure 3. The recombinant fragment pFH 1-7 bound to heparin as shown. Fragment pFH 15-20 showed binding to heparin. Thus pFH contains heparin binding sites in SCR 15-20 and SCR 1-7. Fragment pFH 1-4 showed no binding to heparin indicating that the heparin binding site is located in SCR 5-7.

### Example 6: pFH SCR1-4 Cofactor Activity for human FI in the Cleaveage of human C3b.

The C3b-Cleavage assay was carried out as follows: to a volume of 25µl VBS buffer containing 0.5µg human Factor I and 2.5µg human C3b dilutions of the recombinant proteins in 5µl VBS were added. The reaction mixture was incubated at 37°C for 1 h 30 min with constant rotation of the tubes. 8µl SDS-loading buffer and 3µl DTT 1M were added to the reaction mixture, then denatured at 95°C for 10 min and analysed by SDS-PAGE and subsequent silverstaining or Western blotting. SDS-PAGE was carried out under reducing conditions. Western blotting was carried out using anti-C3b (sheep) first antibody and HRP-conjugated anti-sheep (goat) secondary antibody.

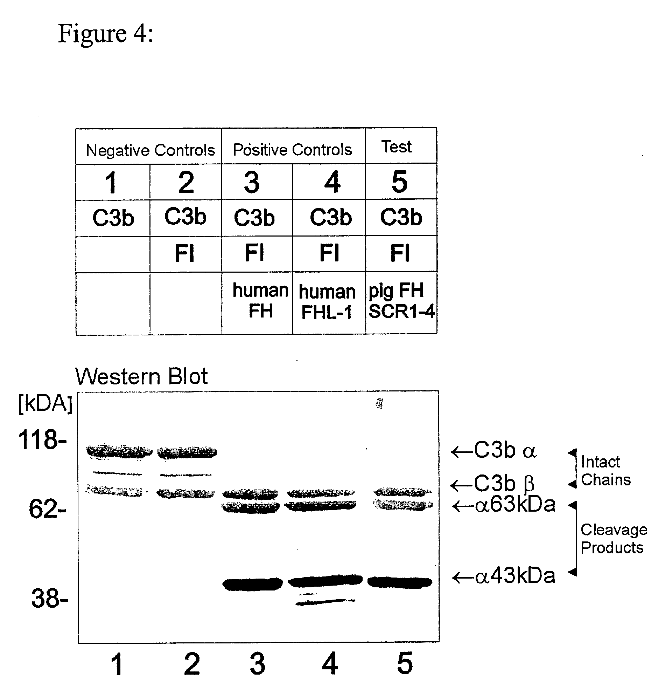

Results are shown in Figure 4. hC3b alone or hC3b and hFI were incubated as negative controls. As positive controls hC3b and hFI were incubated either with hFH or with hFHL-1. Cofactor activity for pFH was analyzed using the pFH SCR1-4 fragment. Reactions were gel separated and used for Western blot and silver staining. Cofactor activity is shown by the cleavage of the α'-chain of C3b to the α63kDa fragment. Blots were incubated with an anti-C3b(sheep) antibody showing strong reactivity for both chains of intact hC3b and for the cleavage fragments. Cleavage products were detectable for all three cofactors used in the assay. pFH SCR 1-4 was shown to display cofactor activity for human FI in the cleavage of human C3b.

### Example 7: Quantitative Comparison of Cleavage Activity of pFH SCR1-4 and human FHL-1.

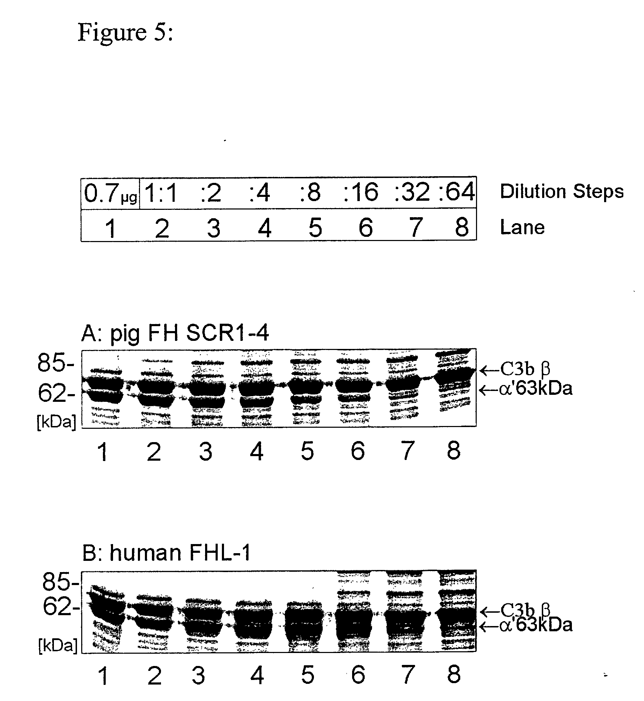

To assess the enzymatic activity of pFH compared to that of the human analogue, a nonradioactive quantitative cleavage assay was used. As a human standard recombinant hFHL-1 was chosen for comparison with the pFH fragment. pFH SCR1-4 was used as the experimental cofactor. The results are shown in Figure 5. 0.7 µg recombinant human FHL-1 and pFH SCR 1-4 in a volume of 5 µl each were used as starting concentration and diluted serially. Each dilution series was then used as cofactors for the cleavage of hC3b by hFI. The results are shown for pFH SCR1-4 (panel A) and hFHL-1 (panel B). Cleavage of the α-chain of human C3b to the α63 kDa fragment could be detected for both cofactors. The titer of the activity was defined as the cofactor dilution step that showed half-maximum cleavage activity of hC3b measured by disappearance of the α63 kDa band. For both the human and the porcine cofactor the half-maximum cleavage activity was detected in dilution steps 5 to 6 (corresponding to dilution 1:8 to 1:16). Half-maximum activity in the cleavage of human C3b differed less than one dilution step for pFH SCR 1-4 and human FHL-1 and thus shows comparable cofactor activity on a quantitative level.

### Reference List

1. Roitt, I., J. Brostoff, and J. Male. *Immunology.* Mosby International Ltd.
2. Pangburn, M. K. and H. J. Muller-Eberhard. 1983. Initiation of the alternative complement pathway due to spontaneous hydrolysis of the thioester of C3. *Ann.N.Y.Acad.Sci.* 421:291-298.
3. Fearon, D. T. and K. F. Austen. 1975. Initiation of C3 cleavage in the alternative complement pathway. *J.Immunol.* 115:1357-1361.
4. Meri, S. and M. K. Pangburn. 1990. Discrimination between activators and nonactivators of the alternative pathway of complement: regulation via a sialic acid/polyanion binding site on factor H. *Proc.Natl.Acad.Sci.U.S.A* 87:3982-3986.
5. Pangburn, M. K., R. D. Schreiber, and H. J. Muller-Eberhard. 1977. Human complement C3b inactivator: isolation, characterization, and demonstration of an absolute requirement for the serum protein betalH for cleavage of C3b and C4b in solution. *J.Exp.Med.* 146:257-270.
6. Weiler, J. M., M. R. Daha, K. F. Austen, and D. T. Fearon. 1976. Control of the amplification convertase of complement by the plasma protein betalH. *Proc.Natl.Acad.Sci.U.S.A* 73:3268-3272.
7. Whaley, K. and S. Ruddy. 1976. Modulation of the alternative complement pathways by beta 1 H globulin. *J.Exp.Med.* 144:1147-1163.
8. Ripoche, J., A. J. Day, T. J. Harris, and R. B. Sim. 1988. The complete amino acid sequence of human complement factor H. *Biochem.J.* 249:593-602.
9. Zipfel, P. F., J. Hellwage, M. A. Friese, G. Hegasy, S. T. Jokiranta, and S. Meri. 1999. Factor H and disease: a complement regulator affects vital body functions. *Mol.Immunol.* 36:241-248.
10. Kuhn, S., C. Skerka, and P. F. Zipfel. 1995. Mapping of the complement regulatory domains in the human factor H-like protein 1 and in factor H1. *J.Immunol.* 155:5663-5670.
11. Zipfel, P. F., T. S. Jokiranta, J. Hellwage, V. Koistinen, and S. Meri. 1999. The factor H protein family. *Immunopharmacology* 42:53-60.
12. Schwaeble, W. and Sim, R. B. Complement Inhibitor. World Intellectual Property Organization PCT/GB97/03275, WO 9823638. 1997. Ref Type: Patent
13. Kristensen, T. and B. F. Tack. 1986. Murine protein H is comprised of 20 repeating units, 61 amino acids in length. *Proc.Natl.Acad.Sci.U.S.A* 83:3963-3967.
14. Soames, C. J., A. J. Day, and R. B. Sim. 1996. Prediction from sequence comparisons of residues of factor H involved in the interaction with complement component C3b. *Biochem.J.* 315 ( Pt 2):523-531.
15. Ish, C., G. L. Ong, N. Desai, and M. J. Mattes. 1993. The specificity of alternative complement pathway-mediated lysis of erythrocytes: a survey of complement and target cells from 25 species. *Scand.J.Immunol.* 38:113-122.
16. Byrne, G. W., K. R. McCurry, M. J. Martin, S. M. McClellan, J. L. Platt, and J. S. Logan. 1997. Transgenic pigs expressing human CD59 and decay-accelerating factor produce an intrinsic barrier to complement-mediated damage. *Transplantation* 63:149-155.
17. Lundwall, A. and G. Eggertsen. 1985. Isolation of human complement factors C3, C5 and H. *J.Immunol.Methods* 81:147-160.
18. Hammer, C. H., G. H. Wirtz, L. Renfer, H. D. Gresham, and B. F. Tack. 1981. Large scale isolation of functionally active components of the human complement system. *J.Biol.Chem.* 256:3995-4006.
19. Kaidoh, T., T. Fujita, Y. Takata, S. Natsuume-Sakai, and M. Takahashi. 1984. Simplified method for purification of mouse beta 1H. *Complement* 1:44-51.
20. Katz, Y. and R. C. Strunk. 1989. IL-1 and tumor necrosis factor. Similarities and differences in stimulation of expression of alternative pathway of complement and IFN-beta 2/IL-6 genes in human fibroblasts. *J.Immunol.* 142:3862-3867.
21. Lemercier, C., N. Julen, M. Coulpier, H. Dauchel, D. Ozanne, M. Fontaine, and J. Ripoche. 1992. Differential modulation by glucocorticoids of alternative complement protein secretion in cells of the monocyte/macrophage lineage. *Eur.J.Immunol.* 22:909-915.
22. Collen, D. and H. K. Gold. 1990. New developments in thrombolytic therapy. *Adv.Exp.Med.Biol.* 281:333-354.
23. Groenewoud, A. F. and J. de Boer. 1994. A report of the eurotransplant randomized multicenter study comparing kidney graft preservation with HTK, UW and EC solutions. HTK study group. *Transpl.Int.* 7 Suppl 1:S479-S480.
24. Andersson, J., R. Larsson, R. Richter, K. N. Ekdahl, and B. Nilsson. 2001. Binding of a model regulator of complement activation (RCA) to a biomaterial surface: surface-bound factor H inhibits complement activation. *Biomaterials* 22:2435-2443.
25. Joziasse, D. H. and R. Oriol. 1999. Xenotransplantation: the importance of the Galalpha1,3Gal epitope in hyperacute vascular rejection. *Biochim.Biophys.Acta* 1455:403-418.
26. Suckfull, M., M. Mudsam, O. Pieske, G. Enders, R. Babic, and C. Hammer. 1994. Immunohistological studies of complement activation after xenogeneic perfusion of a working heart model. *Transpl.Int.* 7:324-328.
27. Forty, J., D. J. White, and J. Wallwork. 1993. Activation of the alternative pathway of complement in hyperacute xenograft rejection of rabbit hearts by human blood. *J.Heart Lung Transplant.* 12:283-286.
28. Lambrigts, D., D. H. Sachs, and D. K. Cooper. 1998. Discordant organ xenotransplantation in primates: world experience and current status. *Transplantation* 66:547-561.
29. Ault, B. H. 2000. Factor H and the pathogenesis of renal diseases. *Pediatr.Nephrol.* 14:1045-1053.
30. Taylor, C. M. 2001. Hemolytic-uremic syndrome and complement factor H deficiency: clinical aspects. *Semin.Thromb.Hemost*. 27:185-190.
31. Homeister, J. W. and B. R. Lucchesi. 1994. Complement activation and inhibition in myocardial ischemia and reperfusion injury. *Annu.Rev.Pharmacol.Toxicol*. 34:17-40.
32. Chakraborti, T., A. Mandal, M. Mandal, S. Das, and S. Chakraborti. 2000. Complement activation in heart diseases. Role of oxidants. *Cell Signal*. 12:607-617.
33. Wintero, A. K., M. Fredholm, and W. Davies. 1996. Evaluation and characterization of a porcine small intestine cDNA library: analysis of 839 clones. *Mamm.Genome* 7:509-517.
34. Kuhn, S. and P. F. Zipfel. 1995. The baculovirus expression vector pBSV-8His directs secretion of histidine-tagged proteins. *Gene* 162:225-229.

### Figure legends

Figure 1: Nucleotide and deduced Amino Acid Sequence of pFH.
   The numbers on the left refer to the nucleotide sequence. Numbers on the right refer to the amino acid sequence starting with the first residue (E-1 SCR1) of the mature protein. Signal peptide and SCRs are marked above the corresponding amino acid. N-glycosilation sites are boxed.
Figure 2: Functional Map of hFH and expressed Fragments of pFH
   Panel (A): The short consensus repeat (SCR) organization of human factor H
   Panel (B): Functional domains mapped to SCRs in human factor H. Heparin Binding: human Factor H heparin/polyanion binding sites; Regulatory Activity: complement-regulatory activity of human Factor H (decay-accelerating and cofactor activity).
   Panel (C): Map of four pFH fragments expressed recombinantly.
   Panel (D): Purified pFH fragments expressed recombinantly. Proteins were separated by SDS-PAGE and visualized by silver staining. Approximate mobility of the molecular weight standard is indicated on the right.
Figure 3: Heparin Binding Characteristics of recombinant Fragments pFH SCR1-4, pFH SCR1-7 and pFH SCR15-20.
   Diluted cell culture supernatants containing the appropriate fragments were applied onto a heparin sepharose column. Heparin columns were washed 8 times. Bound proteins were eluted with a linear salt gradient in 8 elution steps. All collected fractions were analysed on Western blot using anti-pFH (rabbit) antibody. W8 refers to the 8^{th} washing fraction. E2 refers to the 2^{nd} elute fraction.
   pFH SCR1-4: Fragment does not bind to heparin.
   pFH SCR1-7 and pFH SCR 15-20: Fragments bind to heparin.
Figure 4: Qualitative Analysis of pFH Cofactor Activity.
   Non-radioactive cleavage assay reactions were run on SDS-PAGE and visualized by Western blot using anti-C3b (sheep) antibody. Reaction components are shown above every lane in a box. Lane 1 and 2 are negative controls containing hC3b or hC3b and hFI respectively. Lane 3 and 4 are positive controls containing hC3b and hFI and the human cofactors hFH or hFHL-1 respectively. Lane 5 contained recombinant pFH SCR 1-4 as cofactor. Approximate mobility of protein markers in kDa are shown on the left. Approximate mobility of C3b cleavage products are indicated on the right by arrows. Porcine protein pFH acts as a cofactor for human FI in the cleavage of human C3b as shown in lane 5 by the appearance of the hC3b cleavage products α63 and α43.
Figure 5: Quantitative Analysis of pFH Cofactor Activity
   Serial dilutions of pFH SCR 1-4 and human FHL-1 were compared for their cofactor activity. Both recombinant proteins were diluted serially. Initial protein amounts (lane 1) were 0.7 µg for both pFH SCR 1-4 and human FHL-1. Dilution steps and corresponding lanes are shown in the upper box. pFH SCR 1-4 (A) and human FHL-1 (B) were used as cofactors for the cleavage of hC3b by hFI. Reactions were run on SDS-PAGE under denaturing conditions and silver stained. Positions of protein markers in kDa are shown on the left. Approximate mobility of the cleavage products of hC3b is indicated on the right by arrows. pFH is shown to be a quantitative equivalent cofactor for the cleavage of hC3b by hFI as hFHL-1, indicated by similar gradual disappearance of the cleavage product α63 of hC3b.

### Table legends

Figure 6 (Table): Primers used for Sequencing pFH and Construction of pFH Fragments.

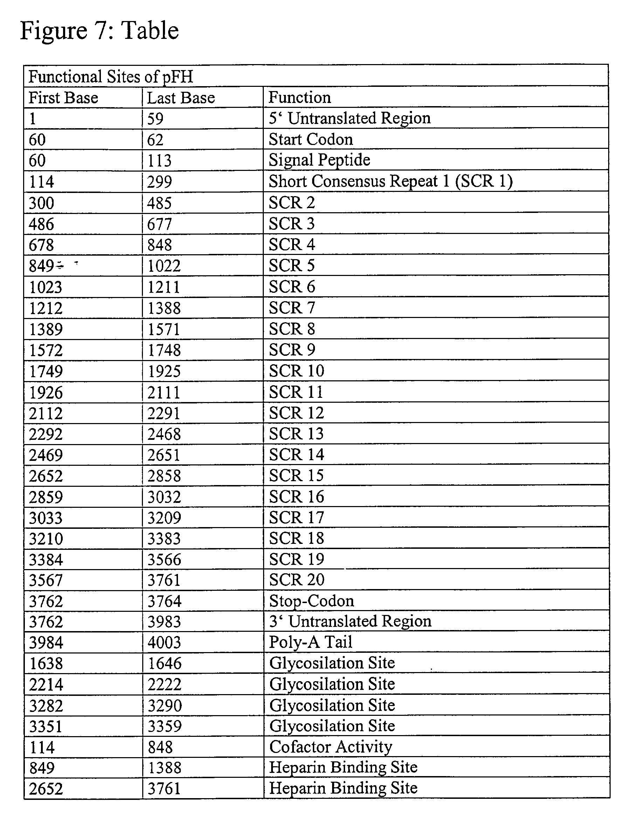

Figure 7 (Table): Functional Sites of pFH. First and last base of the sites and their functions are shown.

## Claims

1. A porcine complement regulator factor H (pFH) protein selected from the group consisting of
(A) mature pFH,
(B) a biologically and / or enzymatically active fragment of said mature pFH, and
(C) a biologically and / or enzymatically active variant or mutant of said mature pFH or fragment thereof.

2. Substantially pure pFH according to claim 1 having the amino acid sequence depicted in SEQ ID NO 2.

3. A pharmaceutical composition comprising a pFH protein according to claim 1.

4. A pFH protein according to claim 1 for use in a method for the prophylactical or therapeutical treatment of the human or animal body.

5. Use of a pFH protein according to claim 1 to control complement activation in vitro or ex vivo.

6. A nucleic acid selected from the group consisting of a nucleic acid coding for a pFH protein according to claim 1 and the corresponding complementary nucleic acid.

7. A DNA according to claim 6 having the nucleotide sequence depicted in SEQ ID NO 1.

8. Use of a nucleic acid according to claim 6 for the preparation of a cell, tissue or animal wherein said cell, tissue or animal expresses or is capable of being caused to express increased amounts of a pFH protein according to claim 1.

9. A vector comprising a nucleic acid sequence coding for a pFH protein according to claim 1.


SEQUENCE LISTING

<110>  Hans-Knöll-Institut für Naturstoff-Forschung e.V.
       Hegasy, Guido
       Zipfel, Peter

<120>  Porcine complement regulator factor H

<130>  EP_02_03

<160>  34

<170>  PatentIn version 3.1

<210>  1
<211>  4003
<212>  DNA
<213>  Pig

<220>
<221>  sig_peptide
<222>  (60)..(113)
<223>

<220>
<221>  CDS
<222>  (114)..(3761)
<223>

<400>  1
cagaacctga cacagtgttg aacactcaac tcctagatgt accctgaacg gtccccaaaa      60

tgagatgtcc agcaaagttt gtgtggctaa tattgtggac tgtttgtgtt gca gaa        116
                                                           Glu
                                                           1

gac tgt aga gaa cct cct cca aga aaa gaa aca gaa att gta tca ggt       164
Asp Cys Arg Glu Pro Pro Pro Arg Lys Glu Thr Glu Ile Val Ser Gly
            5                   10                  15

tcc tgg act gaa caa aca tat cca gaa ggc act cag gct aca tac aaa       212
Ser Trp Thr Glu Gln Thr Tyr Pro Glu Gly Thr Gln Ala Thr Tyr Lys
        20                  25                  30

tgc cgc cct gga tac aga aca ctt ggg agt att gtt atg gtg tgc aag       260
Cys Arg Pro Gly Tyr Arg Thr Leu Gly Ser Ile Val Met Val Cys Lys
    35                  40                  45

gat gga aaa tgg gtg tct ctt cat ccg tcc agg ata tgt cgg aaa aag       308
Asp Gly Lys Trp Val Ser Leu His Pro Ser Arg Ile Cys Arg Lys Lys
50                  55                  60                  65

ccc tgt gga cat cct gga gac aca tcc ttt ggt tct ttc cat ctt gca       356
Pro Cys Gly His Pro Gly Asp Thr Ser Phe Gly Ser Phe His Leu Ala
                70                  75                  80

gta gga act cag ttt gaa tac ggt gcc aag gtt gtt tac aca tgc gat 404
Val Gly Thr Gln Phe Glu Tyr Gly Ala Lys Val Val Tyr Thr Cys Asp
            85                  90                  95

gag ggg tat cag atg ctg ggt gag att aat ttt cgt gaa tgt gat aca 452
Glu Gly Tyr Gln Met Leu Gly Glu Ile Asn Phe Arg Glu Cys Asp Thr
        100                 105                 110

aac gga tgg acc aat aat atc ccc tta tgt gaa gtt gtt aag tgt tta 500
Asn Gly Trp Thr Asn Asn Ile Pro Leu Cys Glu Val Val Lys Cys Leu
    115                 120                 125

ccg gtg aca gaa cca gag aat ggg agg att gtc agt ggt gcc ctg gaa 548
Pro Val Thr Glu Pro Glu Asn Gly Arg Ile Val Ser Gly Ala Leu Glu
130                 135                 140                 145

cca gat caa gaa tac act tac gga cag gtg gtg cag ttc cag tgt aat 596
Pro Asp Gln Glu Tyr Thr Tyr Gly Gln Val Val Gln Phe Gln Cys Asn
                150                 155                 160

gca ggc tac agt ctt gat gga cct aaa caa ata cac tgc tca gca ggt 644
Ala Gly Tyr Ser Leu Asp Gly Pro Lys Gln Ile His Cys Ser Ala Gly
            165                 170                 175

ggt gtt tgg agt gga gac aag ccg aaa tgc gtc gga atc tcc tgc aca 692
Gly Val Trp Ser Gly Asp Lys Pro Lys Cys Val Gly Ile Ser Cys Thr
        180                 185                 190

cca cca aac att tta aat ggg tac cct ata att tca aag gaa att tat 740
Pro Pro Asn Ile Leu Asn Gly Tyr Pro Ile Ile Ser Lys Glu Ile Tyr
    195                 200                 205

aag gca aat gag cga gtt cag tat aaa tgt tca gcg ggt ttt gaa tac 788
Lys Ala Asn Glu Arg Val Gln Tyr Lys Cys Ser Ala Gly Phe Glu Tyr
210                 215                 220                 225

agt gag aga gga gaa acc att tgc acg gca tct gga tgg gcc cca gtt 836
Ser Glu Arg Gly Glu Thr Ile Cys Thr Ala Ser Gly Trp Ala Pro Val
                230                 235                 240

cct tca tgt gta gaa ata acg tgt gat cct cct cat att cca aat ggt 884
Pro Ser Cys Val Glu Ile Thr Cys Asp Pro Pro His Ile Pro Asn Gly
            245                 250                 255

ttt tac aca cct gaa tca aac aga tac aga act gga gac aga atc act 932
Phe Tyr Thr Pro Glu Ser Asn Arg Tyr Arg Thr Gly Asp Arg Ile Thr
        260                 265                 270

tat cac tgc aaa gaa ggc ttt tat cct gag atc cag gga aat gtt gca 980
Tyr His Cys Lys Glu Gly Phe Tyr Pro Glu Ile Gln Gly Asn Val Ala
    275                 280                 285

aga tgc acc ggc aat cat tgg tca cct gct cca aga tgt acc ttg aaa 1028
Arg Cys Thr Gly Asn His Trp Ser Pro Ala Pro Arg Cys Thr Leu Lys
290                 295                 300                 305

cct tgt agc cgt cca gtc ata aaa cat gga aag cta tat tat gac tat 1076
Pro Cys Ser Arg Pro Val Ile Lys His Gly Lys Leu Tyr Tyr Asp Tyr
                310                 315                 320

cgt gga tac ttt cca gca aat gta gga cag tat ttt tac tac tac tgt 1124
Arg Gly Tyr Phe Pro Ala Asn Val Gly Gln Tyr Phe Tyr Tyr Tyr Cys
            325                 330                 335

gat cat aat ttt gtg act ccc tca cgc cgt tcc ggg gac tac ctg acg 1172
Asp His Asn Phe Val Thr Pro Ser Arg Arg Ser Gly Asp Tyr Leu Thr
        340                 345                 350

tgc aaa cgg aat gga tgg tct gca gaa gtc cca tgc ctc aga caa tgt 1220
Cys Lys Arg Asn Gly Trp Ser Ala Glu Val Pro Cys Leu Arg Gln Cys
    355                 360                 365

att ttt gat tat ttg gaa cat gga cac aac tca tat tca gca aaa aaa 1268
Ile Phe Asp Tyr Leu Glu His Gly His Asn Ser Tyr Ser Ala Lys Lys
370                 375                 380                 385

tat tta cag ggt gaa act gta aga gtt cag tgc tat ccc ggc tac agt 1316
Tyr Leu Gln Gly Glu Thr Val Arg Val Gln Cys Tyr Pro Gly Tyr Ser
                390                 395                 400

ctc cca gat aat cag gac acg ctg ctc tgt aca gag aat ggc tgg tct 1364
Leu Pro Asp Asn Gln Asp Thr Leu Leu Cys Thr Glu Asn Gly Trp Ser
            405                 410                 415

cct cct ccc aag tgc atc ctt gtc aaa aca tgt gca aaa tca gat ata 1412
Pro Pro Pro Lys Cys Ile Leu Val Lys Thr Cys Ala Lys Ser Asp Ile
        420                 425                 430

gaa att gag aat gga ttt ttt tct gaa aat ata ttt aca tat ccc tta 1460
Glu Ile Glu Asn Gly Phe Phe Ser Glu Asn Ile Phe Thr Tyr Pro Leu
    435                 440                 445

aat aaa caa aca caa tat aag tgt aaa cca gga tat gta aca gca gat 1508
Asn Lys Gln Thr Gln Tyr Lys Cys Lys Pro Gly Tyr Val Thr Ala Asp
450                 455                 460                 465

ggc gca act tca gga tta atc aca tgt ctg caa agt gga tgg tca gct 1556
Gly Ala Thr Ser Gly Leu Ile Thr Cys Leu Gln Ser Gly Trp Ser Ala
                470                 475                 480

cga cct gtg tgc att aag tcc tgc gat gcg cca cta cct gag aat gcc 1604
Arg Pro Val Cys Ile Lys Ser Cys Asp Ala Pro Leu Pro Glu Asn Ala
            485                 490                 495

aga atc aag agt gat ggc acg tgg ttt aag ctc aat gac aca gtg gac 1652
Arg Ile Lys Ser Asp Gly Thr Trp Phe Lys Leu Asn Asp Thr Val Asp
        500                 505                 510

tat gaa tgc cag caa ggg ttt gaa agc cga aat gga gac act gca gga 1700
Tyr Glu Cys Gln Gln Gly Phe Glu Ser Arg Asn Gly Asp Thr Ala Gly
    515                 520                 525

tcc att gtg tgt ggt gaa gac gga tgg tct gat aag cct gct tgt tac 1748
Ser Ile Val Cys Gly Glu Asp Gly Trp Ser Asp Lys Pro Ala Cys Tyr
530                 535                 540                 545

gaa aga gaa tgt att act cct gga ata aaa agc tat tta gat gct gaa 1796
Glu Arg Glu Cys Ile Thr Pro Gly Ile Lys Ser Tyr Leu Asp Ala Glu
                550                 555                 560

ccc aaa cat aac aaa ttc aaa gtt gga ggc gtg ttg aaa ttc cac tgc 1844
Pro Lys His Asn Lys Phe Lys Val Gly Gly Val Leu Lys Phe His Cys
            565                 570                 575

aga gaa aga cgt gtg atg ctt gga gca gat tca gtt caa tgc tac cac 1892
Arg Glu Arg Arg Val Met Leu Gly Ala Asp Ser Val Gln Cys Tyr His
        580                 585                 590

ttt gga tgg tcc cct aat ctt cca aca tgt aaa gca ggg caa gta aaa 1940
Phe Gly Trp Ser Pro Asn Leu Pro Thr Cys Lys Ala Gly Gln Val Lys
    595                 600                 605

tcc tgt gct cca ccc cct caa ctc ctc aat ggg gaa gtt aaa gaa aca 1988
Ser Cys Ala Pro Pro Pro Gln Leu Leu Asn Gly Glu Val Lys Glu Thr
610                 615                 620                 625

cag aaa gga gaa tat caa cac agt gag gtg gtg gaa tac gtt tgc aat 2036
Gln Lys Gly Glu Tyr Gln His Ser Glu Val Val Glu Tyr Val Cys Asn
                630                 635                 640

cct aga ttt ctg atg aag ggc tct cat aaa att caa tgt gtt gat gga 2084
Pro Arg Phe Leu Met Lys Gly Ser His Lys Ile Gln Cys Val Asp Gly
            645                 650                 655

gaa tgg aca acc ttg cct gtg tgt att gag gag gag cgt acc tgt gga 2132
Glu Trp Thr Thr Leu Pro Val Cys Ile Glu Glu Glu Arg Thr Cys Gly
        660                 665                 670

gat att ccg gac ctc gac cat ggc tat gtc cgg cat tct gcc cct ccc 2180
Asp Ile Pro Asp Leu Asp His Gly Tyr Val Arg His Ser Ala Pro Pro
    675                 680                 685

tat cac cat gga gac tca gtg gag ttc ggc tgc aat gca tca ttt tca 2228
Tyr His His Gly Asp Ser Val Glu Phe Gly Cys Asn Ala Ser Phe Ser
690                 695                 700                 705

ctg gtt gga ccc agg tca atc acc tgt att agt gga aag tgg acc caa 2276
Leu Val Gly Pro Arg Ser Ile Thr Cys Ile Ser Gly Lys Trp Thr Gln
                710                 715                 720

ctt cct cag tgc ttt gca aca gat aaa ctc aag aag tgt aaa gcc tca 2324
Leu Pro Gln Cys Phe Ala Thr Asp Lys Leu Lys Lys Cys Lys Ala Ser
            725                 730                 735

aaa ata ttt gca tct gag gga aac ctg tta gat aag acc gaa ttt gac 2372
Lys Ile Phe Ala Ser Glu Gly Asn Leu Leu Asp Lys Thr Glu Phe Asp
        740                 745                 750

cat aac acc aac caa agt tac aaa tgt cgg gga aaa tca gaa agc aaa 2420
His Asn Thr Asn Gln Ser Tyr Lys Cys Arg Gly Lys Ser Glu Ser Lys
    755                 760                 765

tac tca gtc tgt ata aat gga gta tgg gat ccc aga gta agc tgc aaa 2468
Tyr Ser Val Cys Ile Asn Gly Val Trp Asp Pro Arg Val Ser Cys Lys
770                 775                 780                 785

gaa gaa gta ttg aat tca tgc cca cct cca cct cag att cct aat gct 2516
Glu Glu Val Leu Asn Ser Cys Pro Pro Pro Pro Gln Ile Pro Asn Ala
                790                 795                 800

caa gat atg aca act aca gtg aat tat aag gat gga gaa aaa ata tct 2564
Gln Asp Met Thr Thr Thr Val Asn Tyr Lys Asp Gly Glu Lys Ile Ser
            805                 810                 815

att ctc tgt caa gaa aac tat ata att caa gat gga gaa gaa att gtg 2612
Ile Leu Cys Gln Glu Asn Tyr Ile Ile Gln Asp Gly Glu Glu Ile Val
        820                 825                 830

tgc aaa gat gga aga tgg cag tca gtc cca cgc tgt gtt gaa aaa att 2660
Cys Lys Asp Gly Arg Trp Gln Ser Val Pro Arg Cys Val Glu Lys Ile
    835                 840                 845

cca tgt tct gaa cca cct gaa ata gac cat gga acc att cag tca cct 2708
Pro Cys Ser Glu Pro Pro Glu Ile Asp His Gly Thr Ile Gln Ser Pro
850                 855                 860                 865

ggc tct gca gaa gaa agg aga gaa atg ttt gag ccc aag aaa tat gca 2756
Gly Ser Ala Glu Glu Arg Arg Glu Met Phe Glu Pro Lys Lys Tyr Ala
                870                 875                 880

cat ggc acc agg tta agt tat act tgt gaa gat ggt ttc gag ata tct 2804
His Gly Thr Arg Leu Ser Tyr Thr Cys Glu Asp Gly Phe Glu Ile Ser
            885                 890                 895

gaa aaa gag ata ata tgc cac atg gga aaa tgg agt tct ttg cct cag 2852
Glu Lys Glu Ile Ile Cys His Met Gly Lys Trp Ser Ser Leu Pro Gln
        900                 905                 910

tgt gtg ggt cgt cct tgt gca cca cca cct gag att cag aac ggt gct 2900
Cys Val Gly Arg Pro Cys Ala Pro Pro Pro Glu Ile Gln Asn Gly Ala
    915                 920                 925

gtc gct cag gag aaa gac agc tac ctg tac gga gaa gaa gtt acc tac 2948
Val Ala Gln Glu Lys Asp Ser Tyr Leu Tyr Gly Glu Glu Val Thr Tyr
930                 935                 940                 945

agc tgt gat gaa ggt ttt ggg att gat gga cct gca tct ata aga tgt 2996
Ser Cys Asp Glu Gly Phe Gly Ile Asp Gly Pro Ala Ser Ile Arg Cys
                950                 955                 960

tta gga gaa aaa tgg tcc cat cct cca gaa tgc ata aac acg gat tgt 3044
Leu Gly Glu Lys Trp Ser His Pro Pro Glu Cys Ile Asn Thr Asp Cys
            965                 970                 975

ttt gac tta ccc acc ttc aat gac gct gta gtc ata ggc cca aag aag 3092
Phe Asp Leu Pro Thr Phe Asn Asp Ala Val Val Ile Gly Pro Lys Lys
        980                 985                 990

cca tcg tat aag tca gga gag cag gtg act ttt aaa tgt ctg caa tat 3140
Pro Ser Tyr Lys Ser Gly Glu Gln Val Thr Phe Lys Cys Leu Gln Tyr
    995                 1000                1005

ttt caa ttg gaa gga cct aac acc ata cgg tgt att aag agc aga 3185
Phe Gln Leu Glu Gly Pro Asn Thr Ile Arg Cys Ile Lys Ser Arg
1010                1015                1020

tgg ata gga aag cca gca tgc aga gat gtt tcc tgt gtg aat cca 3230
Trp Ile Gly Lys Pro Ala Cys Arg Asp Val Ser Cys Val Asn Pro
1025                1030                1035

ccc agt gtg gaa aac gct gtt ata cta gat aag agg tca agg tat 3275
Pro Ser Val Glu Asn Ala Val Ile Leu Asp Lys Arg Ser Arg Tyr
1040                1045                1050

caa cct ggt gag aga gca cgt tat gag tgc atc gaa cct tac gcc 3320
Gln Pro Gly Glu Arg Ala Arg Tyr Glu Cys Ile Glu Pro Tyr Ala
1055                1060                1065

ctt tat ggg gat gta gaa gtg atg tgt ttt aat gga agt tgg acc 3365
Leu Tyr Gly Asp Val Glu Val Met Cys Phe Asn Gly Ser Trp Thr
1070                1075                1080

aaa cca cct cag tgc aaa gac tct aaa gga aaa tgt ggg cca ccc 3410
Lys Pro Pro Gln Cys Lys Asp Ser Lys Gly Lys Cys Gly Pro Pro
1085                1090                1095

cca cct ata gac aat gga gac acg acc tca ttc cca tta cca gtc 3455
Pro Pro Ile Asp Asn Gly Asp Thr Thr Ser Phe Pro Leu Pro Val
1100                1105                1110

tat cct ccg gga aca gtc gtc gag tac caa tgc cag tcc tac tat 3500
Tyr Pro Pro Gly Thr Val Val Glu Tyr Gln Cys Gln Ser Tyr Tyr
1115                1120                1125

gaa ctt gag gga agc agc tcc ata aaa tgt gag aat gga cag tgg 3545
Glu Leu Glu Gly Ser Ser Ser Ile Lys Cys Glu Asn Gly Gln Trp
1130                1135                1140

tca gaa cca cca aaa tgc tta gat gca tgt gta gtt tca gaa gaa 3590
Ser Glu Pro Pro Lys Cys Leu Asp Ala Cys Val Val Ser Glu Glu
1145                1150                1155

atg atg aga aaa cat aac ata gag cta aaa tgg aga cca gat aaa 3635
Met Met Arg Lys His Asn Ile Glu Leu Lys Trp Arg Pro Asp Lys
1160                1165                1170

aaa ctt tat tcc aga aca gat gat act att gaa ttt cgg tgt aga 3680
Lys Leu Tyr Ser Arg Thr Asp Asp Thr Ile Glu Phe Arg Cys Arg
1175                1180                1185

caa gga tat tat agg agg acg cca cta cat aca ttc cga gca acc 3725
Gln Gly Tyr Tyr Arg Arg Thr Pro Leu His Thr Phe Arg Ala Thr
1190                1195                1200

tgt cag caa ggc aaa gtg gcc tat cct acg tgt gga taaaacagtg 3771
Cys Gln Gln Gly Lys Val Ala Tyr Pro Thr Cys Gly
1205                1210                1215

gttaaaatgc agtcataaaa ctgaaatatg tacatttact cagagtttgt cattattaat 3831

ccagttctcc atttatttga tcgactatta ttgtttaact catctttatt cagaaatcag 3891

catttgtgtt gaatagtatg tggatgttgt aattataatc tgggaaacca atgaattact 3951

tctttaaagc atctcattaa acttggcaaa tcaaaaaaaa aaaaaaaaaa aa 4003

<210> 2
<211> 1216
<212> PRT

<213>  Pig

<400>  2

Glu Asp Cys Arg Glu Pro Pro Pro Arg Lys Glu Thr Glu Ile Val Ser
1               5                   10                  15

Gly Ser Trp Thr Glu Gln Thr Tyr Pro Glu Gly Thr Gln Ala Thr Tyr
            20                  25                  30

Lys Cys Arg Pro Gly Tyr Arg Thr Leu Gly Ser Ile Val Met Val Cys
        35                  40                  45

Lys Asp Gly Lys Trp Val Ser Leu His Pro Ser Arg Ile Cys Arg Lys
    50                  55                  60

Lys Pro Cys Gly His Pro Gly Asp Thr Ser Phe Gly Ser Phe His Leu
65                  70                  75                  80

Ala Val Gly Thr Gln Phe Glu Tyr Gly Ala Lys Val Val Tyr Thr Cys
                85                  90                  95

Asp Glu Gly Tyr Gln Met Leu Gly Glu Ile Asn Phe Arg Glu Cys Asp
            100                 105                 110

Thr Asn Gly Trp Thr Asn Asn Ile Pro Leu Cys Glu Val Val Lys Cys
        115                 120                 125

Leu Pro Val Thr Glu Pro Glu Asn Gly Arg Ile Val Ser Gly Ala Leu
    130                 135                 140

Glu Pro Asp Gln Glu Tyr Thr Tyr Gly Gln Val Val Gln Phe Gln Cys
145                 150                 155                 160

Asn Ala Gly Tyr Ser Leu Asp Gly Pro Lys Gln Ile His Cys Ser Ala
                165                 170                 175

Gly Gly Val Trp Ser Gly Asp Lys Pro Lys Cys Val Gly Ile Ser Cys
            180                 185                 190

Thr Pro Pro Asn Ile Leu Asn Gly Tyr Pro Ile Ile Ser Lys Glu Ile
        195                 200                 205

Tyr Lys Ala Asn Glu Arg Val Gln Tyr Lys Cys Ser Ala Gly Phe Glu
    210                 215                 220

Tyr Ser Glu Arg Gly Glu Thr Ile Cys Thr Ala Ser Gly Trp Ala Pro
225                 230                 235                 240

Val Pro Ser Cys Val Glu Ile Thr Cys Asp Pro Pro His Ile Pro Asn
                245                 250                 255

Gly Phe Tyr Thr Pro Glu Ser Asn Arg Tyr Arg Thr Gly Asp Arg Ile
            260                 265                 270

Thr Tyr His Cys Lys Glu Gly Phe Tyr Pro Glu Ile Gln Gly Asn Val
        275                 280                 285

Ala Arg Cys Thr Gly Asn His Trp Ser Pro Ala Pro Arg Cys Thr Leu
    290                 295                 300

Lys Pro Cys Ser Arg Pro Val Ile Lys His Gly Lys Leu Tyr Tyr Asp
305                 310                 315                 320

Tyr Arg Gly Tyr Phe Pro Ala Asn Val Gly Gln Tyr Phe Tyr Tyr Tyr
                325                 330                 335

Cys Asp His Asn Phe Val Thr Pro Ser Arg Arg Ser Gly Asp Tyr Leu
            340                 345                 350

Thr Cys Lys Arg Asn Gly Trp Ser Ala Glu Val Pro Cys Leu Arg Gln
        355                 360                 365

Cys Ile Phe Asp Tyr Leu Glu His Gly His Asn Ser Tyr Ser Ala Lys
    370                 375                 380

Lys Tyr Leu Gln Gly Glu Thr Val Arg Val Gln Cys Tyr Pro Gly Tyr
385                 390                 395                 400

Ser Leu Pro Asp Asn Gln Asp Thr Leu Leu Cys Thr Glu Asn Gly Trp
                405                 410                 415

Ser Pro Pro Pro Lys Cys Ile Leu Val Lys Thr Cys Ala Lys Ser Asp
            420                 425                 430

Ile Glu Ile Glu Asn Gly Phe Phe Ser Glu Asn Ile Phe Thr Tyr Pro
        435                 440                 445

Leu Asn Lys Gln Thr Gln Tyr Lys Cys Lys Pro Gly Tyr Val Thr Ala
    450                 455                 460

Asp Gly Ala Thr Ser Gly Leu Ile Thr Cys Leu Gln Ser Gly Trp Ser
465                 470                 475                 480

Ala Arg Pro Val Cys Ile Lys Ser Cys Asp Ala Pro Leu Pro Glu Asn
                485                 490                 495

Ala Arg Ile Lys Ser Asp Gly Thr Trp Phe Lys Leu Asn Asp Thr Val
            500                 505                 510

Asp Tyr Glu Cys Gln Gln Gly Phe Glu Ser Arg Asn Gly Asp Thr Ala
        515                 520                 525

Gly Ser Ile Val Cys Gly Glu Asp Gly Trp Ser Asp Lys Pro Ala Cys
    530                 535                 540

Tyr Glu Arg Glu Cys Ile Thr Pro Gly Ile Lys Ser Tyr Leu Asp Ala
545                 550                 555                 560

Glu Pro Lys His Asn Lys Phe Lys Val Gly Gly Val Leu Lys Phe His
                565                 570                 575

Cys Arg Glu Arg Arg Val Met Leu Gly Ala Asp Ser Val Gln Cys Tyr
            580                 585                 590

His Phe Gly Trp Ser Pro Asn Leu Pro Thr Cys Lys Ala Gly Gln Val
        595                 600                 605

Lys Ser Cys Ala Pro Pro Pro Gln Leu Leu Asn Gly Glu Val Lys Glu
    610                 615                 620

Thr Gln Lys Gly Glu Tyr Gln His Ser Glu Val Val Glu Tyr Val Cys
625                 630                 635                 640

Asn Pro Arg Phe Leu Met Lys Gly Ser His Lys Ile Gln Cys Val Asp
                645                 650                 655

Gly Glu Trp Thr Thr Leu Pro Val Cys Ile Glu Glu Glu Arg Thr Cys
            660                 665                 670

Gly Asp Ile Pro Asp Leu Asp His Gly Tyr Val Arg His Ser Ala Pro
        675                 680                 685

Pro Tyr His His Gly Asp Ser Val Glu Phe Gly Cys Asn Ala Ser Phe
    690                 695                 700

Ser Leu Val Gly Pro Arg Ser Ile Thr Cys Ile Ser Gly Lys Trp Thr
705                 710                 715                 720

Gln Leu Pro Gln Cys Phe Ala Thr Asp Lys Leu Lys Lys Cys Lys Ala
                725                 730                 735

Ser Lys Ile Phe Ala Ser Glu Gly Asn Leu Leu Asp Lys Thr Glu Phe
            740                 745                 750

Asp His Asn Thr Asn Gln Ser Tyr Lys Cys Arg Gly Lys Ser Glu Ser
        755                 760                 765

Lys Tyr Ser Val Cys Ile Asn Gly Val Trp Asp Pro Arg Val Ser Cys
    770                 775                 780

Lys Glu Glu Val Leu Asn Ser Cys Pro Pro Pro Pro Gln Ile Pro Asn
785                 790                 795                 800

Ala Gln Asp Met Thr Thr Thr Val Asn Tyr Lys Asp Gly Glu Lys Ile
                805                 810                 815

Ser Ile Leu Cys Gln Glu Asn Tyr Ile Ile Gln Asp Gly Glu Glu Ile
            820                 825                 830

Val Cys Lys Asp Gly Arg Trp Gln Ser Val Pro Arg Cys Val Glu Lys
        835                 840                 845

Ile Pro Cys Ser Glu Pro Pro Glu Ile Asp His Gly Thr Ile Gln Ser
    850                 855                 860

Pro Gly Ser Ala Glu Glu Arg Arg Glu Met Phe Glu Pro Lys Lys Tyr
865                 870                 875                 880

Ala His Gly Thr Arg Leu Ser Tyr Thr Cys Glu Asp Gly Phe Glu Ile
                885                 890                 895

Ser Glu Lys Glu Ile Ile Cys His Met Gly Lys Trp Ser Ser Leu Pro
            900                 905                 910

Gln Cys Val Gly Arg Pro Cys Ala Pro Pro Pro Glu Ile Gln Asn Gly
        915                 920                 925

Ala Val Ala Gln Glu Lys Asp Ser Tyr Leu Tyr Gly Glu Glu Val Thr
    930                 935                 940

Tyr Ser Cys Asp Glu Gly Phe Gly Ile Asp Gly Pro Ala Ser Ile Arg
945                 950                 955                 960

Cys Leu Gly Glu Lys Trp Ser His Pro Pro Glu Cys Ile Asn Thr Asp
                965                 970                 975

Cys Phe Asp Leu Pro Thr Phe Asn Asp Ala Val Val Ile Gly Pro Lys
            980                 985                 990

Lys Pro Ser Tyr Lys Ser Gly Glu Gln Val Thr Phe Lys Cys Leu Gln
        995                 1000                1005

Tyr Phe Gln Leu Glu Gly Pro Asn Thr Ile Arg Cys Ile Lys Ser
    1010                1015                1020

Arg Trp Ile Gly Lys Pro Ala Cys Arg Asp Val Ser Cys Val Asn
    1025                1030                1035

Pro Pro Ser Val Glu Asn Ala Val Ile Leu Asp Lys Arg Ser Arg
    1040                1045                1050

Tyr Gln Pro Gly Glu Arg Ala Arg Tyr Glu Cys Ile Glu Pro Tyr
    1055                1060                1065

Ala Leu Tyr Gly Asp Val Glu Val Met Cys Phe Asn Gly Ser Trp
    1070                1075                1080

Thr Lys Pro Pro Gln Cys Lys Asp Ser Lys Gly Lys Cys Gly Pro
    1085                1090                1095

Pro Pro Pro Ile Asp Asn Gly Asp Thr Thr Ser Phe Pro Leu Pro
    1100                1105                1110

Val Tyr Pro Pro Gly Thr Val Val Glu Tyr Gln Cys Gln Ser Tyr
    1115                1120                1125

Tyr Glu Leu Glu Gly Ser Ser Ser Ile Lys Cys Glu Asn Gly Gln
    1130                1135                1140

Trp Ser Glu Pro Pro Lys Cys Leu Asp Ala Cys Val Val Ser Glu
    1145                1150                1155

Glu Met Met Arg Lys His Asn Ile Glu Leu Lys Trp Arg Pro Asp
    1160                1165                1170

Lys Lys Leu Tyr Ser Arg Thr Asp Asp Thr Ile Glu Phe Arg Cys
    1175                1180                1185

Arg Gln Gly Tyr Tyr Arg Arg Thr Pro Leu His Thr Phe Arg Ala
    1190                1195                1200

Thr Cys Gln Gln Gly Lys Val Ala Tyr Pro Thr Cys Gly
    1205                1210                1215

<210> 3
<211> 21
<212> DNA
<213> Artificial Sequence

<220>
<223> Sequencing primer

<220>
<221> misc_feature
<222> (1)..(21)
<223>

<400> 3
gcagaacctg acacagtgtt g                                                21

<210> 4
<211> 20
<212> DNA
<213> Artificial Sequence

<220>
<223> Sequencing primer

<220>
<221> misc_feature
<222> (1)..(20)
<223>

<400> 4
ggaggattgt cagtggtgcc                                                  20

<210> 5
<211> 21
<212> DNA
<213> Artificial Sequence

<220>
<223> Sequencing primer

<220>
<221> misc_feature
<222> (1)..(21)
<223>

<400>  5
gcatctgata cccctcatcg c                                                21

<210>  6
<211>  20
<212>  DNA
<213>  Artificial Sequence

<220>
<223>  Sequencing primer
<220>
<221>  misc_feature
<222>  (1)..(20)
<223>

<400>  6
ggtttttaca cacctgaatc                                                  20

<210>  7
<211>  20
<212>  DNA
<213>  Artificial Sequence

<220>
<223>  Sequencing primer

<220>
<221>  misc_feature
<222>  (1)..(20)
<223>

<400>  7
gattcaggtg tgtaaaaacc                                                  20

<210>  8
<211>  21
<212>  DNA
<213>  Artificial Sequence

<220>
<223>  Sequencing primer

<220>
<221>  misc_feature
<222>  (1)..(21)
<223>

<400>  8
gttcagtgct atcccggcta c                                                21

<210>  9
<211>  21
<212>  DNA
<213>  Artificial Sequence

<220>
<223>  Sequencing primer

<220>
<221>  misc_feature
<222>  (1)..(21)
<223>

<400>  9
gtagccggga tagcactgaa c                                                21


<210>  10
<211>  20
<212>  DNA
<213>  Artificial Sequence

<220>
<223>  Sequencing primer

<220>
<221>  misc_feature
<222>  (1)..(20)
<223>

<400>  10
tgcgccacta cctgagaatg                                                  20


<210>  11
<211>  20
<212>  DNA
<213>  Artificial Sequence

<220>
<223>  Sequencing primer

<220>
<221>  misc_feature
<222>  (1)..(20)
<223>

<400>  11
tcaaaccctt gctggcattc                                                  20


<210>  12
<211>  25
<212>  DNA
<213>  Artificial Sequence

<220>
<223>  Sequencing primer

<220>
<221>  misc_feature
<222>  (1)..(25)
<223>

<400>  12

ggggaaaatc agaaagcaaa tactc 25

<210> 13
<211> 25
<212> DNA
<213> Artificial Sequence

<220>
<223> Sequencing primer

<220>
<221> misc_feature
<222> (1)..(25)
<223>

<400> 13
gagtatttgc tttctgattt tcccc 25

<210> 14
<211> 20
<212> DNA
<213> Artificial Sequence

<220>
<223> Sequencing primer

<220>
<221> misc_feature
<222> (1)..(20)
<223>

<400> 14
tgcatgtgta gtttcagaag 20

<210> 15
<211> 20
<212> DNA
<213> Artificial Sequence

<220>
<223> Sequencing primer

<220>
<221> misc_feature
<222> (1)..(20)
<223>

<400> 15
cttctgaaac tacacatgca 20

<210> 16
<211> 20
<212> DNA
<213> Artificial Sequence

<220>
<223>  Sequencing primer

<220>
<221>  misc_feature
<222>  (1)..(20)
<223>

<400>  16
tgccaagttt aatgagatgc                                                    20


<210>  17
<211>  22
<212>  DNA
<213>  Artificial Sequence

<220>
<223>  Sequencing primer

<220>
<221>  misc_feature
<222>  (1)..(22)
<223>

<400>  17
cataggccca aagaagccat cg                                                 22


<210>  18
<211>  22
<212>  DNA
<213>  Artificial Sequence

<220>
<223>  Sequencing primer

<220>
<221>  misc_feature
<222>  (1)..(22)
<223>

<400>  18
cgatggcttc tttgggccta tg                                                 22


<210>  19
<211>  20
<212>  DNA
<213>  Artificial Sequence

<220>
<223>  Sequencing primer

<220>
<221>  misc_feature
<222>  (1)..(20)
<223>

<400>  19

cttgtgaaga tggtttcgag                                                    20

<210>  20
<211>  20
<212>  DNA
<213>  Artificial Sequence

<220>
<223>  Sequencing primer

<220>
<221>  misc_feature
<222>  (1)..(20)
<223>

<400>  20
ctcgaaacca tcttcacaag                                                    20

<210>  21
<211>  22
<212>  DNA
<213>  Artificial Sequence

<220>
<223>  Sequencing primer

<220>
<221>  misc_feature
<222>  (1)..(22)
<223>

<400>  21
caaaccacct cagtgcaaag ac                                                 22

<210>  22
<211>  22
<212>  DNA
<213>  Artificial Sequence

<220>
<223>  Sequencing primer

<220>
<221>  misc_feature
<222>  (1)..(22)
<223>

<400>  22
gtctttgcac tgaggtggtt tg                                                 22

<210>  23
<211>  20
<212>  DNA
<213>  Artificial Sequence

<220>
<223>  Sequencing primer

<220>
<221>  misc_feature
<222>  (1)..(20)
<223>

<400>  23
tgaaccacct gaaatagacc                                                    20


<210>  24
<211>  20
<212>  DNA
<213>  Artificial Sequence

<220>
<223>  Sequencing primer

<220>
<221>  misc_feature
<222>  (1)..(20)
<223>

<400>  24
tttgggattg atggacctgc                                                    20


<210>  25
<211>  20
<212>  DNA
<213>  Artificial Sequence

<220>
<223>  Sequencing primer

<220>
<221>  misc_feature
<222>  (1)..(20)
<223>

<400>  25
agcacgttat gagtgcatcg                                                    20


<210>  26
<211>  23
<212>  DNA
<213>  Artificial Sequence

<220>
<223>  Sequencing primer

<220>
<221>  misc_feature
<222>  (1)..(23)
<223>

<400>  26

ccaccccctc aactcctcaa tgg                                                23

<210>  27
<211>  25
<212>  DNA
<213>  Artificial Sequence

<220>
<223>  Sequencing primer

<220>
<221>  misc_feature
<222>  (1)..(25)
<223>

<400>  27
gggtccaacc agtgaaaatg atgca                                              25

<210>  28
<211>  32
<212>  DNA
<213>  Artificial Sequence

<220>
<223>  fragment construction primer

<220>
<221>  misc_feature
<222>  (1)..(32)
<223>

<400>  28
gcggccgctg aagactgtag agaacctcct cc                                      32

<210>  29
<211>  31
<212>  DNA
<213>  Artificial Sequence

<220>
<223>  fragment construction primer

<220>
<221>  misc_feature
<222>  (1)..(31)
<223>

<400>  29
aactgcagga agactgtaga gaacctcctc c                                       31

<210>  30
<211>  31
<212>  DNA
<213>  Artificial Sequence

<220>
<223>  fragment construction primer

<220>
<221>  misc_feature
<222>  (1)..(31)
<223>

<400>  30
ggaattctac acatgaagga actggggccc a                                    31


<210>  31
<211>  31
<212>  DNA
<213>  Artificial Sequence

<220>
<223>  fragment construction primer

<220>
<221>  misc_feature
<222>  (1)..(31)
<223>

<400>  31
ggaattcgac aaggatgcac ttgggaggag g                                    31


<210>  32
<211>  35
<212>  DNA
<213>  Artificial Sequence

<220>
<223>  fragment construction primer

<220>
<221>  misc_feature
<222>  (1)..(35)
<223>

<400>  32
gcggccgctg aaaaaattcc atgttctgaa ccacc                                35


<210>  33
<211>  33
<212>  DNA
<213>  Artificial Sequence

<220>
<223>  fragment constuction primer

<220>
<221>  misc_feature
<222>  (1)..(33)
<223>

<400>  33

cccgggtcca cacgtaggat aggccacttt gcc                                33

<210>  34
<211>  34
<212>  DNA
<213>  Artificial Sequence

<220>
<223>  fragment construction primer

<220>
<221>  misc_feature
<222>  (1)..(34)
<223>

<400>  34
ggaattctcc acacgtagga taggccactt tgcc                               34